# EUROPEAN PATENT SPECIFICATION

(11) **EP 2 734 542 B1**
(45) Date of publication and mention of the grant of the patent: **26.12.2018**
(21) Application number: 12740115.6
(22) Date of filing: 18.07.2012
(51) Int. Cl.: C07K 14/78, G01N 33/53, G01N 33/68, C07K 7/00

(54) **PATHOLOGY BIOMARKER ASSAY**
PATHOLOGISCHER BIOMARKERTEST
DOSAGE DE BIOMARQUEUR PATHOLOGIQUES

(30) Priority: 20.07.2011 US 201113187205
(43) Date of publication of application: 28.05.2014
(60) Divisional application: 17172678.9
(73) Proprietor: Nordic Bioscience A/S, 2730 Herlev (DK)
(72) Inventor: VEIDAL, Sanne S., DK-3650 Ølstykke (DK); KARSDAL, Morten A., DK-2100 Copenhagen Ø (DK); LEEMING, Diana J., DK-2450 Copenhagen SV (DK); BARASCUK, Natasha, DK-2300 Copenhagen S (DK); SKJØT-ARKIL, Helene, DK-2300 Copenhagen S (DK); VASSILIADIS, Efstathios, DK-2610 Rødovre (DK)
(74) Representative: Beck Greener
(86) International application number: PCT/EP2012/064090
(87) International publication number: WO 2013/011056

(56) References cited:
- EP-A1- 1 676 602
- WO-A1-2010/031822
- WO-A2-2007/089303
- WO-A2-2008/021290
- WO-A2-2009/059972
- WO-A2-2010/046443
- US-A- 5 811 268
- US-A1- 2010 209 940
- BARASCUK NATASHA ET AL: "QUANTIFICATION OF MATRIX METALLOPROTEINASE - MEDIATED DEGRADATION OF BIGLYCAN IN BILE DUCT LIGATION RAT MODEL OF LIVER FIBROSIS", HEPATOLOGY, vol. 52, no. 4, suppl. S, 1 October 2010 (2010-10-01), page 669A, XP002690181,
- LEEMING D J ET AL: "INVESTIGATING THE TISSUE TURNOVER PROFILE IN LIVER FIBROSIS BY NOVEL BIOCHEMICAL MARKERS OF EXTRACELLULAR MATRIX REMODELING", JOURNAL OF HEPATOLOGY, vol. 54, no. Suppl. 1, 1 March 2011 (2011-03-01), page S135, XP002690173, 46TH ANNUAL MEETING OF THE EUROPEAN-ASSOCIATION-FOR-THE-STUDY-OF-THE- LIVER (EASL); BERLIN, GERMANY; MARCH 30 -APRIL 03, 2011 ISSN: 0168-8278
- B. HÖGEMANN ET AL: "Expression of Biglycan, Decorin and Proteoglycan-100/CSF-1 in Normal and Fibrotic Human Liver", PATHOLOGY, RESEARCH AND PRACTICE, vol. 193, no. 11-12, 1 January 1997 (1997-01-01), pages 747-751, XP55049024, ISSN: 0344-0338, DOI: 10.1016/S0344-0338(97)80052-0
- MARIAN AJ ET AL: "BIOMARKERS OF CARDIAC DISEASE", EXPERT REVIEW OF MOLECULAR DIAGNOSTICS, FUTURE DRUGS, LONDON, GB, vol. 4, 1 January 2004 (2004-01-01), pages 805-820, XP001246855, ISSN: 1473-7159, DOI: 10.1586/14737159.4.6.805
- ZHENG P-S ET AL: "Versican G3 domain promotes blood coagulation through suppressing the activity of tissue factor pathway inhibitor-1", JOURNAL OF BIOLOGICAL CHEMISTRY, AMERICAN SOCIETY FOR BIOCHEMISTRY AND MOLECULAR BIOLOGY, vol. 281, no. 12, 24 March 2006 (2006-03-24), pages 8175-8182, XP003021626, ISSN: 0021-9258, DOI: 10.1074/JBC.M509182200
- SANDY JOHN D ET AL: "Versican V1 proteolysis in human aorta in vivo occurs at the Glu441-Ala442 bond, a site that is cleaved by recombinant ADAMTS-1 and ADAMTS-4", JOURNAL OF BIOLOGICAL CHEMISTRY, AMERICAN SOCIETY FOR BIOCHEMISTRY AND MOLECULAR BIOLOGY, vol. 276, no. 16, 20 April 2001 (2001-04-20), pages 13372-13378, XP002456446, ISSN: 0021-9258, DOI: 10.1074/JBC.M009737200
- VEIDAL S S ET AL: "MMP mediated type V collagen degradation (C5M) is elevated in ankylosing spondylitis", CLINICAL BIOCHEMISTRY, ELSEVIER INC, US, CA, vol. 45, no. 7, 9 February 2012 (2012-02-09), pages 541-546, XP028416577, ISSN: 0009-9120, DOI: 10.1016/J.CLINBIOCHEM.2012.02.007 [retrieved on 2012-02-21]
- P. MYDEL ET AL: "Neutrophil Elastase Cleaves Laminin-332 (Laminin-5) Generating Peptides That Are Chemotactic for Neutrophils", JOURNAL OF BIOLOGICAL CHEMISTRY, vol. 283, no. 15, 1 January 2008 (2008-01-01), pages 9513-9522, XP055049319, ISSN: 0021-9258, DOI: 10.1074/jbc.M706239200

## Description

The present invention relates to assays for biomarkers useful in the diagnosis of various diseases including fibrosis diseases and prognosis of its development, including biomarkers indicative of the risk of developing disease, e.g. the risk of developing a fibrosis after a chronic injury. Biomarkers for inflammatory diseases such as ankylosing spondylitis are also described as are biomarkers for cardiovascular diseases (CVD).

In particular, according to the present invention, biomarkers relating to degradation fragments of Collagen type I, III, IV, V, and VI, elastin, C-reactive protein, ApoE, lumican, LAMC1, LAMB1, LAMA5 and proteoglycans including Biglycan, Decorin, Versican, and Perlecan are found to be useful.

Fibrotic diseases (including those listed in Table 1) are a leading cause of morbidity and mortality, e.g. cirrhosis with 800,000 death per year worldwide¹.

**Table 1. Different fibrotic diseases²**

| Tissue | Examples of Causes |
|---|---|
| Liver | Viral hepatitis |
| | Schistosomiasis |
| | Steatohepatitis (Alcoholic or non-alcoholic) |
| Lung | Idiopathic pulmonary fibrosis (IPF) |
| | Systemic sclerosis (Scleroderma) |
| Kidney | Nephrogenic systemic fibrosis (NSF) |
| | Diabetes |
| | Untreated hypertension |
| Heart | Heart attack |
| | Hypertension |
| | Atherosclerosis |
| | Restenosis |
| Eye | Macular degeneration, retinal and vitreal retinopathy |
| Skin | Systemic sclerosis and scleroderma, keloids, hypertrophic scars, burns, genetic factors NFS |
| Pancreas | Autoimmune/hereditary causes |
| Intestine | Crohn's disease/inflammatory bowl disease |
| Brain | Alzheimer's disease, AIDS |
| Bone marrow | Cancer, ageing |
| Multi-organ fibrosis | Surgical complications, chemotherapeutic drug-induced fibrosis, radiation-induced fibrosis, mechanical injuries |

A 'fibrotic disease' is any disease giving rise to fibrosis, whether as a main or a secondary symptom.

Fibrosis is the end result of chronic inflammatory reactions induced by a variety of stimuli including persistent infections, autoimmune reactions, allergic responses, chemical insults, radiation, and tissue injury. Fibrosis is characterized by the accumulation and reorganization of the extracellular matrix (ECM). Despite having obvious etiological and clinical distinctions, most chronic fibrotic disorders have in common a persistent irritant that sustains the production of growth factors, proteolytic enzymes, angiogenic factors, and fibrogenic cytokines, which together stimulate the deposition of connective tissue elements, especially collagens and proteoglycans, which progressively remodel and destroy normal tissue architecture^{3, 4}. Despite its enormous impact on human health, there are currently no approved treatments that directly target the mechanisms of fibrosis⁵.

The key cellular mediator of fibrosis is the myofibroblast, which when activated serves as the primary collagen-producing cell.

### Inflammatory conditions

Ankylosing Spondylitis (AS) is a form of chronic inflammation of the spine and the sacroiliac joints. Over time, chronic inflammation of the spine can lead to a complete cementation of the vertebrae(110), a process with a molecular pathology that still remains to be investigated and fully understood. AS is also a systemic disease, affecting other tissues throughout the body including inflammation in or injury to other joints, as well as to organs, such as eyes, heart, lungs, and kidneys(111). The inflammation related processes in AS, involving multiple tissues seems to involve molecular processes including increased MMP activity and collagen deposition, seen in most types of connective tissue turnover.

Extracellular matrix (ECM) remodeling is a key process of tissue homeostasis(112, 113). Specific proteolytic activities are a prerequisite for a range of cellular functions and interactions within the ECM(114). These specific activities are precisely coordinated under normal physiological situations, with a specified sequence of events resulting in controlled tissue turnover. In pathological situations, with special emphasis on connective tissue diseases, the normal repair-response relationship is disturbed (115), leading to excessive remodeling and tissue turnover. The consequence of this ECM remodeling is the release of a range of degradation products of proteins, neoepitopes, generated by the proteases expressed locally in the pathologically affected area. These protein degradation fragments may serve as molecular biomarker targets, as they are more specific for the tissue of origin compared to their intact origins(116).

Endopeptidases, such as matrix metalloproteinases (MMPs), play a major role in the degradation of ECM proteins as collagens and proteoglycans(117, 118). In particular, in connective tissue diseases such as fibrosis, MMP-2 and -9 have been shown to be highly up-regulated(119-121). Recently, neoepitope-based biochemical markers found in urine and serum have received increased attention for their diagnostic and prognostic potential(116). In particular for slow progressing diseases, such as osteoporosis and osteoarthritis, bone resorption and cartilage degradation markers, based on type I and II collagen degradation products respectively, have been studied extensively(122).

### Extracellular Matrix (ECM)

Fibrogenesis is a dynamic process involving complex cellular and molecular mechanisms that usually originates from tissue injury ⁶. Fibrogenesis is the result of an imbalance in normal ECM regulation that alters the concentration of macromolecules leading to increased tissue size and density, with progressively impaired function. These macromolecules are mainly fibrous proteins with structural and adhesive functions, such as collagens and proteoglycans.

### Collagen

Collagens are widely distributed in the human body, i.e. ∼ 30% of the protein mass in the human body is composed of collagens. Collagens are responsible for the structural integrity of the ECM of most connective tissues. The ECM content results from a fine balance between synthesis and degradation tightly controlled through regulation of gene expression and protein secretion, but also through endogenous protease inhibition and protein degradation by metalloproteinases and cysteine proteases ⁷⁻⁹. Table 2 lists the major collagen types with their major tissue distribution.

**Table 2. Major collagen types and their tissue distribution.**

| Collagen type | Tissue distribution |
|---|---|
| I | Most connective tissues |
| II | Cartilage, vitreous humor |
| III | Extensible connective tissues, e.g. liver, skin, lung, vascular system |
| IV | Basement membranes |
| V | Tissues containing collagen I |
| VI | Most connective tissues |
| VII | Skin, bladder, oral mucosa, umbilical cord, amnion |
| VIII | Many tissues, especially endothelium |
| XIII | Endothelial cells, skin, eye, heart, skeletal muscle |
| XIV | Vessel, bone, skin, cartilage, eye, nerve, tendon, uterus |
| XXI | Vessel, heart, stomach, kidney, skeletal muscle, placenta |

Type I collagen is the most abundant collagen and is found in most connective tissues. It is especially important for the structure of bone and skin where the major collagenous components are type I and III collagens ¹⁰.

Collagen type I and III are the major components of liver and lung in a 1:1 ratio in healthy tissue. In addition, collagen type IV and VI are found in the basement membranes in most tissues. The most common localization of type V collagen is within the characteristic collagen fibrils, in association with the collagen type I and III ¹⁰.

Some collagens have a restricted tissue distribution: for example, type II, which is found almost exclusively in cartilage ¹¹.

During fibrogenesis the net amount of collagens increases¹²⁻¹⁴. Table 3 shows by way of example the collagen increase during liver fibrosis.

**Table 3. Changes of the composition of collagen from normal to cirrhotic human liver ¹⁵.**

| Collagen type | Chains | Collagen normal liver (mg/g) | Collagen cirrhotic liver (mg/g) | Times increased | Distribution normal liver (%) | Distribution cirrhotic liver (%) |
|---|---|---|---|---|---|---|
| I | α₁(I) | | 16 | 8 | 37 | 42 |
| | α₂(I) | 2 | | | | |
| III | α₁(III) | 2 | 8 | 4 | 37 | 21 |
| IV | α₁(IV) | 0.5 | 7 | 14 | 9 | 18 |
| | α₂(IV) | | | | | |
| V | α₁(V) | 0.9 | 7 | 8 | 17 | 18 |
| | α₂(V) | | | | | |
| | α₃(V) | | | | | |
| VI | α₁(VI) | 0.01 | 0.1 | 10 | 0.2 | 0.3 |
| | α₂(VI) | | | | | |

Type V collagen has been documented to be critically important for the formation of collagen fibrils(123), exemplified by an almost virtual lack of collagen fibril formation in the col5a1-/- mice. In alignment, the heterozygous mice, was associated with a 50% reduction in fibril number and dermal collagen content. This indicates a central role for type V collagen in the life dependent regulation of fibrillogenesis, suggesting this collagen type to be of pivotal interest in many connective diseases. However, there is still a conceptual lack of understanding of the role of type V collagen turnover in connective diseases that may in part me be due to the technical inabilities for investigation of collagen type V degradation and turnover. Interesting, very recently a limited diverse set of proteins were found to bind type V collagen, beginning to elucidate the molecular function of this molecule in more details, of which MMP-2 was one of them(124). In addition to the molecular characterization, more evidence is emerging that type V collagen directly affects different cellular phenotypes by inducing dynamic motility and other cellular activities, suggesting that this proteins may be more than a passive component of the ECM (125, 126). In direct support of this, we recently described a very strong correlation to liver fibrosis with the formation of type V collagen in two separate animal models of liver fibrosis(127), suggesting a central role of type V collagen formation in excessive tissue turnover.

Type V collagen is a fibril-forming collagen, together with type I, II, III and XI(11), and is formed as heterotrimers of three different α-chains (α1, α2, α3). It typically forms heterofibrils with type I and III collagens and contributes to the organic bone matrix, corneal stroma and the interstitial matrix of muscles, liver, lungs and placenta(128). Type V collagen mutation results in a range of connective tissue diseases, of which the Ehlers-Danlos syndrome (EDS) the best described. EDS is a heterogeneous group of heritable disorders characterized by joint hypermobility, skin changes (e.g. hyperextensibility, thinness and fragility). The disease can be divided into different subtypes, EDS 1 & II. EDS types I and II are characterized by atrophic scars, skin hyper extensibility and joint laxity. It is evident that both subtypes result from mutations in the COL5A1 and COL5A2 genes that encode two of the polypeptide chains of type V collagen(129-131). This highlights the importance of type V collagen in connective tissues diseases.

### Elastin

Elastin is a protein present in many connective tissues, primarily those that are elastic. It has a very high content of the amino acids glycine, valine, alanine, and proline, and has a molecular weight of 64 to 66 kDa. It is organised in an irregular or random coil conformation made up of 830 amino acids. Elastin is made by linking many soluble tropoelastin protein molecules, in a reaction catalyzed by lysyl oxidase, to make a massive insoluble, durable cross-linked array.

Elastin serves an important function in arteries as a medium for pressure wave propagation to help blood flow and is particularly abundant in large elastic blood vessels such as the aorta. Elastin is also very important in the lungs, elastic ligaments and the skin.

Despite much efforts devoted to the understanding of elastin synthesis and turnover, neo-epitopes originating from the proteolytic cleavage of this matrix molecules have until now not been associated with disease development in fibrosis.

### Vimentin

Vimentin is a member of the intermediate filament family of proteins. Intermediate filaments are an important structural feature of eukaryotic cells. They, along with microtubules and actin microfilaments, make up the cytoskeleton. Although most intermediate filaments are stable structures, in fibroblasts, vimentin exists as a dynamic structure. This filament is used as a marker for mesodermally derived tissues, and as such has been used used as an immunohistochemical marker for sarcomas.

Hertig and coworkers (Hertig et al., J Am Soc Nephrol. 2008 Aug;19(8):1584-91) investigated if epithelial-to-mesenchymal transition in renal tubular epithelial cells of subjects with chronic allograft nephropathy could predict the progression of fibrosis in the allograft and measured vimentin expression in 83 biopsies from these. They did find an association between elevated vimentin expression and the intestinal fibrosis score at 1 year after surgery.

In another study of hepatic fibrosis, Meriden and colleagues (Meriden et al., Clin Gastro & Hepatol 2010; 8:289-296) found a significant association between vimentin expression (in biopsies obtained at F0 stage) and fibrosis progression, with elevated levels predicting rapid progression of the hepatic fibrosis.

Accordingly, we wanted to investigate if circulating fragments of vimentin could serve as sensitive and specific biomarkers of fibrosis.

### Proteoglycans

Proteoglycans are a diverse group of macromolecules, which covalently link a variable number of glycosaminoglycan (GAG) side chains to a core protein ¹⁶. These GAGs are polymers of disaccharide repeats (e.g. N-acetyl glucosamine or N-acetyl galactosamine), which are acidic (negatively charged) due to hydroxyl, carboxylated and sulfated side groups on the disaccharide units. This makes them highly hydrophilic, thus aiding the diffusion of water and positive ions (e.g. sodium from extracellular fluids) ¹⁷. Furthermore, GAGs have the ability to form non-covalent links with for example hyaluronic acid chains to form even larger molecular complexes ¹⁶. Table 4 lists the most studied proteoglycans associated with connective tissue.

**Table 4. Proteoglycans of the extracellular matrix of connective tissue**

| Group | Proteoglycans | Origin | Function |
|---|---|---|---|
| Large extracellular proteoglycans (*aggregating and hyaluronan-binding)* | Aggrecan ¹⁸ | Articular cartilage chondrocytes, intervertebral disc, nasal cartilage | Extracellular matrix stability (hyaluronan binding) |
| | Versican ^{19, 20} | Connective tissue: fibroblast, keratinocytes, smooth muscle cells, mesangial cells | Cell-cell and cell-matrix interactions Binding of sugars in Ca-dependent manner |
| | Neurocan ²¹ | Nervous tissue | Binds to neural cell adhesion molecules |
| | Brevican ²² | Nervous tissue | Extracellular matrix stability |
| Small Leucine-rich proteoglycans (*collagen-binding)* | Decorin ²³ | Connective tissue, cartilage, bone | Binds to and connect collagen molecules (matrix stabilization and thickness) Organogenesis Binding of TGFβ |
| | Biglycans ²⁴ | Capillary endothelium, skin (keratinocytes), epithelium of kidney | Cell differentiation Binds and connect collagen fibrils |
| | Fibromodulin ¹⁷ | Connective tissue, bone, cartilage | Regulate orientation of collagen fibers |
| | Lumican ²³ | Cornea, muscle, cartilage, kidney, lung, intestine | Controls spacing and thickness of collagen fibers |
| Cell-associated proteoglycans | Serglycins ²⁵ | Widely distributed to endothelium - intercellular compartments | Hemopoietic cell differentiation Adhesion and activation of lymphoid cells |
| | Syndecans ²⁶ | Widely distributed - often cell membrane bound | Binds collagens, fibronectin, thrombospondin, tenascin and bFGF |
| | Betaglycan ²⁷ | Widely distributed | TGFβ receptor and signaling Possible reservoir of TGFβ |
| Basement membrane proteoglycans | Perlecan ²⁸ | All basement membranes | Selective barrier for macromolecules Cell-adhesion |

### C-REACTIVE PROTEIN

C-reactive protein (CRP) is an acute phase serum protein produced by the liver in response to different clinical conditions such as, inflammation, infection, or trauma²⁹. The production of CRP is induced by cytokines such as IL-6, released from the affected or damaged tissues. The physiological role of CRP is yet unknown and discussions on its pro- or anti-inflammatory actions are ongoing.

### APOLIPOPROTEIN E

Apolipoprotein E (APOE) is a class of apolipoprotein found in the chylomicron (large lipoprotein particles) and intermediate-density lipoprotein particles that binds to specific receptors on liver cells and peripheral cells. It is essential for the normal catabolism of triglyceride-rich lipoprotein constituents. APOE is 299 amino acids long and transports lipoproteins, fat-soluble vitamins, and cholesterol into the lymph system and then into the blood. It is synthesized principally in the liver, but has also been found in other tissues such as the brain, kidneys, and spleen. APOE is essential for the normal catabolism of triglyceride-rich lipoprotein constituents. APOE was initially recognized for its importance in lipoprotein metabolism and cardiovascular disease.

### ELASTIN

Elastin is the extracellular matrix molecule responsible for resilience of tissues and was first thought to be restricted to that role. It is now established that elastin degradation may lead to the production of bioactive peptides influencing cell chemotaxis, cell proliferation, and proteases synthesis in a broad panel of normal and tumor cells.

### LAMC1, LAMA2, LAMB1, and LAMA5

Laminins, a family of extracellular matrix glycoproteins, are the major noncollagenous constituent of basement membranes. They have been implicated in a wide variety of biological processes including cell adhesion, differentiation, migration, signaling, neurite outgrowth and metastasis. Laminins are composed of 3 non-identical chains: laminin alpha, beta and gamma (formerly A, B1, and B2, respectively) and they form a cruciform structure consisting of 3 short arms, each formed by a different chain, and a long arm composed of all 3 chains. Each laminin chain is a multidomain protein encoded by a distinct gene. Several isoforms of each chain have been described. Different alpha, beta and gamma chain isomers combine to give rise to different heterotrimeric laminin isoforms which are designated by Arabic numerals in the order of their discovery. The biological functions of the different chains and trimer molecules are largely unknown, but some of the chains have been shown to differ with respect to their tissue distribution, presumably reflecting diverse functions in vivo.

LAMC1 (formerly LAMB2) is the laminin subunit gamma-1.

LAMA2 is the laminin subunit alpha-2

LAMB1 is the laminin subunit beta-1

LAMA5 is the laminin subunit alpha-5

### Proteases

The imbalance between synthesis and degradation of ECM during fibrogenesis, results from conversion of the low-density subendothelial matrix into matrix rich in interstitial collagens. The increase in collagen and proteoglycans may be due to one or both of (1) a decrease in protein production and (2) impaired protein degradation, and hence less matrix degradation. The decreased protein degradation has recently received increased attention. In the regulation of this process matrix metalloproteinases (MMPs) and their tissue inhibitors (TIMPs) play important roles, as well as other proteases and their inhibitors, such as cystein proteases and the cystatins.

### MMPs

MMPs are a large group of endopeptidases, capable of degrading most if not all components of the ECM. Presently, more than 25 MMPs have been found. MMPs are characterized by an active site containing a metal atom, typically zinc, and are secreted as zymogens. Different MMPs are expressed in different tissues. In Table 5 MMPs in the liver are shown.

**Table 5. MMPs in the liver³⁰⁻³²**

| Family | Protease | Source | Substrate |
|---|---|---|---|
| Collagenases | MMP-1 | HSC | I, II, III, VII, VIII, X, gelatin |
| | MMP-8 | Neutrophil | I, II, III, V, VII, X, gelatin |
| | MMP-13 | HSC, MFB, KC | I, II, III, VII, X, gelatin |
| Stromelysins | MMP-3 | HSC | III, IV, V, IX, X, XI, gelatin, laminin, fibronectin, proteoglycans, glycoproteins, elastin, pro-MMP-1/13 |
| | MMP-10 | HSC | III, IV, V, gelatin, elastin, aggrecan |
| | MMP-11 | HC | PAI-1, week activity against matrix proteins |
| Gelatinases | MMP-2 | HSC, MBF | I, II, III, IV, V, VII, X, XI, gelagin, elastin, laminin |
| | MMP-9 | KC, HSC, HC | I, II, III, IV, V, VII, X, XI, gelagin, elastin, laminin |
| | MMP-7 | HSC | Entactin, gelatin, elastin, fibronectin, vitronectin, laminin, fibrinogen |
| Metalloelastase | MMP-12 | Macrophages | Elastin, gelatins, IV, laminin, fibronectin, entactin, vitronectin, proteoglycan, myelin basic protein, α1-antitripsin |
| MT-MMPs | MMP-14 | HSC, MFB, KC | I, II, III, gelatin, fibronectin, vitronectin, laminin, fibrinogen, pro-MMP-2, pro-MMP-13 |
| | MMP-15 | HC, BDEC | Pro-MMP-2, fibronectin, tenascin, laminin, aggrecan, perlecan |

TIMPs block MMPs' proteolytic activity by binding in a substrate- and tissue- specific manner to MMP and membrane-type 1 metalloproteinase in a trimolecular complex (Table 6). During fibrosis TIMP levels increase dramatically, and MMP levels increase modestly or remain relatively static (except MMP-2) which in all gives a decrease in degradation of collagens.

**Table 6. TIMPs in the liver³¹**

| Name | Sources | Metalloproteinase inhibited |
|---|---|---|
| TIMP-1 | HSC, MFB, KC, HC | Pro-MMP-9, MMP-1, MMP-2, MMP-3, MMP-13 |
| TIMP-2 | KC, HSC | MT-MMP-1, MT-MMP-2, proMMP-2, MMP-3, MMP-13, MMP-7 |
| TIMP-3 | HC | MT-MMP-1, MT-MMP-2, TACE, MMP-13 |

### Fibroblast activation protein

Fibroblast Activation Protein alpha subunit (FAPa or FAP, alpha) is an integral membrane gelatinase belonging to the serine protease family. FAPa is the alpha subunit and DPP4 (CD26) the beta subunit of a heterodimeric membrane-bound proteinase complex also known as 170 kDa Melanoma Membrane Gelatinase, Integral Membrane Serine Proteinase and Seprase. Some cells make only FAPa homodimers, some only DPP4 homodimers. The monomer is inactive. FAP, alpha is selectively expressed in reactive stromal fibroblasts of epithelial cancers, granulation tissue of healing wounds, and malignant cells of bone and soft tissue sarcomas³³. This protein is thought to be involved in the control of fibroblast growth or epithelial-mesenchymal interactions during development, tissue repair, and epithelial carcinogenesis. It has been shown that expression of FAP increase with the stage of fibrosis^{34, 35}.

### ADAMTS

ADAMTS (A Disintegrin And Metalloproteinase with Thrombospondin Motifs) is a family of peptidases, and until now 19 members of this family have been identified in humans. Known functions of the ADAMTS proteases include processing of procollagens and von Willebrand factor as well as cleavage of aggrecan, versican, brevican and neurocan. They have been demonstrated to have important roles in connective tissue organization, coagulation, inflammation, arthritis, angiogenesis and cell migration.

### Cathepsins

There are approximately a dozen members of the cathepsins family of proteases, which are distinguished by their structure, catalytic mechanism, and which proteins they cleave. Most of the members become activated at the low pH found in lysosomes. Thus, the activity of this family lies almost entirely within those organelles (although there are many exceptions). Such as cathepsin K which (among other activities) works extracellularly after secretion by osteoclasts in bone resorption.

Cathepsin K (CAT K) and Cathepsin S (CAT S) are both cysteine proteases.

### Fibrosis biomarkers

A number of biochemical markers have been suggested for fibrotic diseases, although not specific product of the disease. In Table 7 is an example of biochemical markers of liver fibrosis used in clinical trial. In addition there are a lot of examples of biomarkers of other fibrotic diseases^{12, 36-42}.

**Table 7 summarizes some of the known markers of liver fibrosis.**

| **Biomarker** | **Parameters** | **Chronic liver disease** | **Reference** |
|---|---|---|---|
| **One parameter** | | | |
| CRP | | NASH | 43 |
| Hyaluronan | | HCV | 44-47 |
| IGF-I | | HCV | 48 |
| Leptin | | HCV | 49 |
| PIIIP | | HCV | 50 |

| **Several parameters** | **Parameters** | **Chronic liver disease** | **References** |
|---|---|---|---|
| MP3 | PIIINP, MMP1 | HCV | 51, 52 |
| Zheng et al index | HA, PIIICP, PIIINP, Laminin, C-IV | Chronic hepatitis | 53 |
| Lebensztjen et al index | Laminin-2, C-IV, MMP2, MMP9-TIMP1 index | HBV | 54 |
| | Tenascin, hyaluronana, Colalegn VI, TIMP-1 | HBV | 55 |
| Tsochatzis et al index | Leptin, adiponectin, resistin | HCV, HBC, NASH | 56 |
| Patel et al index | Hyaluronan, TIMP-1, α₂-macroglobulin | HCV | 57 |
| | TIMP-1, tenascin, collagen IV, PIIINP, MMP2, laminin, Hyaluronan | NASH | 58 |
| Forns-index (76, 77) | Age, platelet count, γGT, cholesterol | HCV HIV/HCV | 51, 59-62 |
| FibroTest (76, 78) | Haptoglobin, α₂-macroglobulin, apolipoprotein A1, γGT, bilirubin | HCV HIV/HCV NAFLD NAFLD in diabetes patients | 45, 51, 60, 61, 63-75 |
| Actitest | FibroTest + ALT | HCV | 65, 76-78 |
| APRI (Wai-index) | AST, platelet count | HIV/HCV HCV NAFLD | 45, 51, 60, 61, 64, 66, 79-87 |
| Hepascore | Bilirubin, γGT, hyaluronan, α₂-macroglobulin, age, gender | HCV HIV/HCV | 51, 61, 64, 66, 88 |
| FIB-4 | Platelet count, AST, ALT, age | HIV/HCV | 61, 83 |
| SHASTA | Hyaluronan, albumin, AST | HIV/HCV | 61 |
| Fibroindex | FORN+APRI | HCV | 89 |
| Fibrometer test | Platelet count, prothrombin index, AST, α₂-macroglobulin, hyaluronan, urea, age | HIV/HCV HCV NAFLD | 51, 61, 64, 66, 81 |
| NFSA | Age, hyperglycaemia, body mass index, platelets, albumin, AST/ALT | NAFLD | 81 |
| Ultrasound + APRI | | HCV | 82 |
| Metwally et al index | Platelet count, albumin, AST, history of blood transfusion, HBV core antibody | HCV | 90 |
| Mohamadnejad et al index | Age, HBV DNA levels, alkaline phosphatase, albumin, platelet counts, AST | HCV | 91 |
| FibroSpect II | Hyaluronan, TIMP-1, α₂-macroglobulin | HCV | 85, 92, 93 |
| Stepwise combination algorithms | Combination of APRI and Fibrotest | HCV | 94 |
| Imbert-Bismut index | α₂ macroglobulin, AST, ALT γGT, total bilirubin, albumin, α₁ globulin, α₂ globulin, β globulin, γ globulin, apolipoprotein A₁ | HCV | 95 |
| Nunes et al | Age, Platelets, INR, CD4, AST/ALT, Hyaluronan, YKL-40, PIIINP | HCV/HIV HCV | 96 |
| Fibroscan +++ | Fibroscan, Fibrotest, APRI, | HCV | 97 |

US5387504 describes the neo-epitope VDIPEN released by the action of stromelysin at the aggrecan site N₃₄₁ -F₃₄₂ and an RIA assay employing a monoclonal antibody specific for this neo-epitope. More generally the use of monospecific antibodies specific for fragments of aggrecan, generated by specific stromelysin cleavage are described. Elevations of stromelysin occur in osteoarthritis, rheumatoid arthritis, atherosclerotic lesions, gout, inflammatory bowel disease (IBD), idiopathic pulmonary fibrosis (IPF), certain cancers, joint injuries, and numerous inflammatory diseases. Stromelysin is reported to be elevated in idiopathic pulmonary fibrosis, and it is alleged that the assay can be conducted on blood or other biological fluids to detect stromelysin cleavage products of aggrecan and that quantitation of such fragments can be used diagnostically in respect of IPF as well as other conditions. However, no evidence for this is provided and there have to our knowledge been no subsequent publications validating this prediction. Such RIA assays have been commercially available for many years and no reports of their successful use in diagnosing or monitoring any fibrotic disease have appeared.

United States Patent 7,225,080 discloses a method for diagnosis of an inflammatory, a fibrotic or a cancerous disease in a patient by measuring the values of at least four biochemical markers selected from the group consisting of α2-macroglobulin, AST (aspartate aminotransferase), ALT (alanine aminotransferase), GGT (gammaglutamyl transpeptidase), γ-globulin, total bilirubin, albumin, α1-globulin, α2-globulin, haptoglobin, β-globulin, apoA1, IL-10, TGF-β1, apoA2, and apoB in the serum or plasma of said patient, and subsequently combining said values in order to determine the presence of liver fibrosis and/or liver necroinflammatory lesions in said patient. The patent does not teach the quantitative measurement of peptide fragment carrying neo-epitopes generated during fibrotic disease.

United States Patent 6,060,255 describes a method for diagnosing the degree of liver fibrosis, comprising the steps of measuring the concentration of type IV collagen high molecular weight form in a sample using an antibody that specifically binds to type IV collagen, and relating the measurement to the degree of liver fibrosis. Again, no use is made of neo-epitopes produced by proteolytic enzymes acting in the body. The sample is actually digested with pepsin, which may obscure the natural pattern of collagen cleavage in the sample.

United States Patent 4,628,027 (Gay) discloses the production of antibodies specific for connective tissue proteins and, more particularly, the production of monoclonal antibodies by fused cell hybrids against human collagens and enzymes involved in collagen degradation. The use of monoclonal antibodies against connective tissue proteins to establish the collagen profile of histological, cytological and biological fluid samples is described. However, the patent does not describe the measurement of connective tissue proteins based on the binding of antibodies to neo-epitopes on said connective tissue proteins.

Guañabens N et al, J Bone Miner Res, 1998 ⁹⁸ evaluated the bone turnover markers N-telopeptide of type I collagen (NTX), C-telopeptide of type I collagen (CTX) and N-terminal pro-peptide of collagen type I (PINP) in patients with primary biliary cirrhosis, a disease with increased hepatic fibrosis. The level of NTX, CTX and PINP were elevated in patients compared to controls and correlated with the histological stage of the disease. The antibodies employed in the NTX were raised against a cathepsin K cleaved site in the N-terminal of collagen type I and are dependent on the neoepitope YDGKGVG↓ SEQ ID NO 302. The antibodies employed in the CTX were raised against a cathepsin K cleaved site in the C-terminal of collagen type I and are dependent on the neoepitope EKAHDGGR↓ SEQ ID NO 303. These markers are located in telopeptides of collagen type I and not in the internal part (the triple helical part) of collagen type I. The monoclonal antibodies employed for the PINP assay were raised against an internal epitope in the PINP sequence which is not a neo-epitope.

Møller S et al, Gut., 1999 ⁹⁹ demonstrated that the C-terminal cross linked telopeptide of type I collagen (ICTP) was elevated in alcoholic cirrhosis patients compared to controls. The study described showed that a biochemical marker can reflect hepatic fibrosis. The ICTP polyclonal antibody has been raised against trypsin and collagenase cleaved collagen type I. However, the antibodies are not binding to a neo-epitope.

Rosen HN et al, Calcif Tissue Int, 2004 ¹⁰⁰ assessed the bone turnover markers N-telopeptide of type I collagen (NTX) and C-telopeptide of type I collagen (CTX) in women receiving hormone replacement treatment (HRT). In the study it was observed that the bone turnover markers decreased with treatment. The antibodies employed in the NTX were raised against a cathepsin K cleaved site in the N-terminal of collagen type I and are dependent on the neoepitope YDGKGVG↓ SEQ ID NO 302. The antibodies employed in the CTX were raised against a cathepsin K cleaved site in the C-terminal of collagen type I and are dependent on the neoepitope EKAHDGGR↓ SEQ ID NO 303. In contrast to the present invention, these antibodies were used for evaluation of bone metabolism and not fibrosis.

Lein M et al, Eur Urol, 2007 ¹⁰¹ evaluated the use of the neo-epitope specific bone turnover markers N-telopeptide of type I collagen (NTX) and C-telopeptide of type I collagen (CTX) in prostate cancer patients receiving zoledronic acid. In the study it was observed that the bone turnover markers decreased with treatment. The antibodies employed in the NTX were raised against a cathepsin K cleaved site in the N-terminal of collagen type I and are dependent on the neoepitope YDGKGVG↓ SEQ ID NO 302. The antibodies employed in the CTX were raised against a cathepsin K cleaved site in the C-terminal of collagen type I and are dependent on the neoepitope EKAHDGGR↓ SEQ ID NO 303. In contrast to the present invention, these antibodies were used for evaluation of the bone metabolism during invasion of bone metastases and not fibrosis.

PIIINP has been used in a number of studies to assess the severity of fibrotic disease ¹⁰², in patients with skin fibrosis following severe burn trauma ¹⁰³, for disease progression in noncirrhotic primary biliary cirrhosis ¹⁰⁴, in primary biliary cirrhosis and chronic viral hepatitis C ¹⁰⁵.

PIIINP and ICTP were measured in patients with fibrosis of the myocardium ¹⁰⁶.

Many reports combine a set of biochemical markers to improve the predictive value of the biochemical index. Eleven different serum markers were measured in 205 patients with fibrotic staging from F0 to F4, and the most informative markers were alpha2 macroglobulin, alpha2 globulin (or haptoglobin), gamma globulin, apolipoprotein A1, gamma glutamyltranspeptidase, and total bilirubin ¹⁰⁷. An index of these markers had a negative predictive value (100% certainty of absence of F2, F3, or F4) was obtained for scores ranging from zero to 0.10 (12% [41] of all patients), and high positive predictive value (>90% certainty of presence of F2, F3, or F4) for scores ranging from 0.60 to 1.00 (34% [115] of all patients).

WO2010/115749 discloses numerous neoepitopes of some of the proteins described above as fibrosis biomarkers and WO2009/059972 discloses numerous neoepitopes of some of the proteins described above as biomarkers of cardiovascular disease.

US 2010/209940 discloses methods of diagnosis or of quantitation of fibrosis comprising conducting an immunoassay to measure neo-epitope containing protein fragments naturally present in a biofluid sample, and associating an elevation of said measure in said patient above a normal level with the presence or extent of fibrosis.

Marian et al. ("Biomarkers of Cardiac Disease", Expert Rev. Mol. Diagn. 4(6), 805-520 (2004)) discusses selected biomarkers for coronary atherosclerosis, acute coronary syndromes and heart failure.

However, in none of the above mentioned reports is it suggested that measurements of peptide fragments based on antibodies binding to neo-epitopes as now claimed might be useful for the assessment of patients with fibrotic disease or inflammatory disease.

The present invention now provides a method of bioassay comprising, conducting an immunoassay to measure neo-epitope containing protein fragments naturally present in a patient biofluid sample, wherein said immunoassay is conducted by a method comprising:
contacting protein fragments naturally present in said sample with an immunological binding partner reactive with a neo-epitope formed by cleavage of a protein by a proteinase and measuring the extent of binding of peptide fragments to said immunological binding partner to measure therein protein fragments comprising said neo-epitope, wherein said immunological binding partner is specifically reactive with the following sequence at the N terminal of a peptide: KTFGKM

Preferably, the detection of binding is quantative.

The method may include associating an elevation of said measure in said patient above a normal level with the presence of fibrosis, or inflammatory disease. Preferably, the amount of binding is compared to control values established for populations of healthy individuals and of individuals characterised by a fibrotic disease or by an inflammatory condition.

Optionally, an elevated result in an immunoassay according to this invention is associated with skin fibrosis, lung fibrosis, or liver fibrosis or cardiovascular disease. Optionally, an elevated result in an immunoassay according to the invention may be associated with an inflammatory condition, such as ankylosing spondylitis. The method may comprise the preliminary step of obtaining a patient biofluid sample.

Said immunological binding partner has specific binding affinity for peptide fragments comprising the N-terminal neoepitope.

Specific reactivity with or immunological affinity for a neo-epitope will imply that the relevant immunological binding partner is not reactive with intact protein from which the neo-epitope derives. Preferably, said immunological binding partner is not reactive with a neo-epitope sequence if the sequence is prolonged past the respective cleavage site. This will imply that the peptides measured terminate with the designated sequence.

The term 'immunological binding partner' as used herein includes polyclonal and monoclonal antibodies and also specific binding fragments of antibodies such as Fab or F(ab')₂. Thus, said immunological binding partner may be a monoclonal antibody or a fragment of a monoclonal antibody having specific binding affinity.

Preferably, the neo-epitope sequence to which the immunological binding partner binds is not found in any other protein or is not found in any of the other proteins to which the method of the invention relates.

The result of an assay according to the invention may be combined with one or more other measured biomarkers to form a composite index of diagnostic or prognostic value.

Generally, all previously known immunoassay formats can be used in accordance with this invention including heterogeneous and homogeneous formats, sandwich assays, competition assays, enzyme linked assays, radio-immune assays and the like. Thus, optionally, said method is conducted as a competition immunoassay in which said immunological binding partner and a competition agent are incubated in the presence of said sample and the competition agent competes with the peptide fragments in the sample to bind to the immunological binding partner.

Said competition agent may be (1) a synthetic peptide derived from the sequence of versican or a competition agent derived from (2) a purified native protein named above cleaved by proteases to reveal said neo-epitope.

One suitable method could be a competition immunoassay using monoclonal antibodies or antibody binding fragments binding to neo-epitopes of an above named protein. Appropriately selected synthetic peptides coated onto the solid surface of a microtitre plate could compete with the sample for binding to the monoclonal antibodies or binding fragments. Alternatively, purified, native protein fragments carrying the neo-epitope recognised by the monoclonal antibody or binding fragment could be used on the solid surface. Yet another alternative is to immobilise the monoclonal antibody or binding fragment on the solid surface and then co-incubate the sample with a synthetic peptide appropriately linked to a signal molecule, e.g. horseradish peroxidase or biotin.

The sample may be a sample of serum, blood, or plasma or an other type, e.g. fibrotic tissue biopsy.

Assays may be conducted as sandwich assays using a first immunological binding partner specifically reactive with a said neo-epitope and a second immunological binding partner reactive with the relevant protein to which the neo-epitope belongs. Optionally, said second immunological binding partner is directed to a second neo-epitope of the same protein.

The development of monoclonal antibodies recognising specific neo-epitopes is as follows. This can be achieved by immunising mice with synthetic peptides originating from the amino acid sequence of molecules of a named protein (including the sequences listed above or sequences terminating therein), fusing the spleen-cells from selected mice to myeloma cells, and testing the monoclonal antibodies for binding to neo-epitopes on relevant synthetic peptides. Specificity for neo-epitopes can be ensured by requiring reactivity with a synthetic peptide and a lack of reactivity with either a C-prolongated form of the immunising peptide (for a C-terminal neo-epitope) or an N-terminal prolongated form of the immunising peptide (for an N-terminal neo-epitope). Antibodies for neo-epitopes may also be evaluated to establish a lack of binding capacity to native protein. Alternatively, specificity for a neo-epitope can be ensured by requiring the reactivity of the antibody to be negatively dependent on the presence of biotin or other functional groups covalently linked to one of the terminal amino acids.

The following describes kits which can be used conveniently for carrying out the methods described above. Such kits may include (1) a microtitre plate coated with synthetic peptide; (2) a monoclonal antibody or antibody binding fragment reactive with said synthetic peptide; and (3) a labelled anti-mouse IgG immunoglobulin. Alternatively, such kits may include (1) a microtitre plate coated with purified native protein fragments; (2) a monoclonal antibody recognising a neo-epitope on the relevant fragments and reactive with said purified protein fragments; and (3) a labelled anti-mouse IgG immunoglobulin. Alternatively, such kits may include (1) a microtitre plate coated with streptavidin; (2) a synthetic peptide linked to biotin; (3) a monoclonal antibody recognising a neo-epitope on protein fragments and reactive with said synthetic peptide; and (4) a labelled anti-mouse IgG immunoglobulin. Yet another alternative could be kits including (1) a microtitre plate coated with streptavidin; (2) a synthetic peptide linked to biotin; (3) a monoclonal antibody recognising a neo-epitope on relevant protein fragments (and reactive with said synthetic peptide) and conjugated to horseradish peroxidase.

The assays described herein are useful in the diagnosis of fibrosis or inflammatory conditions in patients. In addition, the tests are useful for the assessment of disease progression, and the monitoring of response to therapy. The immunological binding partners disclosed herein may also be used in immunostaining to show the presence or location of the disclosed protein cleavage products.
Figure 1 in panels A and B shows the results of investigating the specificity of a monoclonal antibody;
Figure 2 shows the results of investigating the amount of Collagen V neo-epitope bearing fragments in human serum from ankylosing spondylitis patients.
Figure 3 shows a standard curve obtained in Example 5;
Figure 4 shows peptide release measured from *ex vivo* cultures in Example 5;
Figure 5 shows further peptide measurements obtained in Example 5;
Figure 6 shows in panels (a) to (d) further measurements obtained in Example 5;
Figure 7 shows further peptide measurements in serum obtained in Example 5;
Figure 8 shows peptide in serum measured in Example 6;
Figure 9 shows a test of antibody specificity described in Example 7;
Figure 10 shows plasma levels of peptide measured in Example 7;
Figure 11 shows ROC curves obtained in Example 7;
Figure 12 shows ELISA results obtained in Example 8;
Figure 13 shows peptide measurements relating to ankylosing spondylitis obtained in Example 8; and
Figure 14 shows results obtained in Example 9.

### Example 1: Identification of Type V Collagen fragments by enzyme cleavage

### Reagents

All reagents used for the experiments were standard high-quality chemicals from companies such as Merck (Whitehouse Station, NJ, USA) and Sigma Aldrich (St.Louis, MO, USA). The synthetic peptides used for monoclonal antibody production were purchased from the Chinese Peptide Company, Beijing, China.

### In vitro cleavage

Purified type V collagen from human placenta (cat. no. ab7537, Abcam, Cambridge, UK) was cleaved with pro-MMP-2 or pro-MMP-9 (cat. no. 444213; 444231; Calbiochem, Merck, Whitehouse Station, NJ, USA). Fifty µg MMP-2 or MMP-9 was activated with 20 µl 1 mM 4-aminophenylmercuric acetate (APMA) in dimethyl sulfoxide and incubated at 37°C for 3 hours. Type V collagen was delivered dissolved in 0.5M acetic acid. To facilitate MMP cleavage, the protein was dialyzed for two days to remove the acetic acid. The liquid was filtered to remove proteins below 10 kDa (Microcon Ultracel YM-10, cat. no. 42407, Millipore, Billerica, MA, USA). Each MMP cleavage was performed separately by mixing 100 µg type V collagen and 1 µg of either MMP-2 or MMP-9 in MMP buffer (100 mM Tris-HCl, 100 mM NaCl, 10 mM CaCl₂, 2 mM Zn acetate, pH 8.0). As control, 100 µg of collagen was mixed with MMP buffer alone. The solutions were incubated for 2 hours at 37°C. The cleavage reaction was stopped using 50 µM ethylenediaminetetraacetic acid (EDTA) to a final concentration of 1µM. Cleavage was verified by visualization using the SilverXpress® Silver Staining Kit (cat. no. LC6100, Invitrogen, Carlsbad, Ca, USA) according to the manufacturer's instructions.

### Peptide identification

Peptide fragments in the *in vitro* cleaved samples were identified using liquid chromatography (LC) coupled to electrospray ionization (ESI) tandem mass spectrometry (LC-MS/MS). LC-MS samples were ultra-filtrated to remove proteins above 10 kDa, the pH was adjusted to 2.0 using formic acid, and a 4 µL sample was analyzed by LC-MS/MS. LC was performed on a nanoACQUITY UPLC BEH C₁₈ column (Waters, Milford, MA, USA) using a formic acid/acetonitrile gradient. MS and MS/MS were performed on a Synapt High Definition Mass Spectrometry quadruple time of flight MS (QUAD-TOF; Waters, Milford, MA, USA), with an acquisition range of 350-1600 m/z in MS and 50-2000 m/z, in MS/MS. The software "ProteinLynx Global SERVER (PLGS)" (Waters, Milford, MA, USA) was used to analyze spectra and generate peak lists. To identify peptides, MS and MS/MS data was searched against a type V collagen (FASTA) protein database using the Mascot 2.2 (Matrix Science, Boston, MA, USA) software with the ESI-QUAD-TOF settings and carbamidomethyl (C), oxidation of methionine (M), oxidation of lysine (K) and oxidation of proline (P) as variable modifications.

The six amino acids in the N- or C-terminal of the peptides identified by MS were regarded as a neo-epitope generated by the protease in question. All protease-generated sequences were analyzed for homology and distance to other cleavage sites and tested for homology using NPS@: network protein sequence analysis (Combet C, Blanchet C, Geourjon C, Deleage G. NPS@:network protein sequence analysis. Trends Biochem Sci 2000; 25: 147-50).

### Example 2: Development of an ELISA assay for identified fragments

### Peptide conjugation

The peptide conjugation was performed using the Maleidide Activated Immunogen Conjugation Kit (Sigma-Aldrich, MO, USA). Briefly, the cysteine-containing immunogenic neo-epitope (HMGREGREGE-GGC, 400 µl peptide at 5 mg/ml) with one free sulfhydryl (-SH) group was mixed in conjugation buffer with the maleimide-activated ovalbumin (OVA) (180 µl OVA at 10 mg/ml) as a carrier protein with an available maleimide group that could react with sulfhydryl-containing peptides and incubated for 2 hours at room temperature. Conjugated products were cleared of EDTA and sodium azide by desalting or dialysis for two days. For the biotin-conjugated peptides, the biotin-conjugated lysine was added in the solid-phase peptide synthesis procedure.

### Monoclonal antibody development

4-6 weeks-old Balb/C mice were immunized subcutaneously with about 200 µl emulsified antigen and 50 µg of the neo-epitope CO5-MMP (HMGREGREGE-GGC-OVA). Consecutive immunizations were performed at 2-week intervals until stable sera titer levels were reached in Freund's incomplete adjuvant. Blood samples were collected from the 2^{nd} immunization. At each blood sampling, the serum titer was determined and the mouse with highest anti-serum titer was selected for fusion. After the 4^{th} immunization, this mouse was rested for 1 month and then boosted intravenously with 50 µg CO5-MMP in 100 µl 0.9% sodium chloride solution three days before isolation of the spleen for cell fusion.

### Fusion and antibody screening

The fusion procedure performed as described by Gefter et al¹³². Briefly, mouse spleen cells were fused with SP2/0 myeloma fusion partner cells. The hybridoma cells were cloned using a semi-solid medium method and transferred into 96-well microtiter plates for further growth and incubated in a CO₂-incubater. Standard limited dilution was used to promote monoclonal growth. Supernatants were screened using an indirect ELISA with streptavidin-coated microtitre plates and HMGREGREGE-K-Biotin as a capture peptide.

### Characterization of clones

Native reactivity and peptide binding of the monoclonal antibodies was evaluated by displacement of native samples (human/rat/mouse serum, plasma and urine) in a preliminary ELISA using 10 ng/mL biotinylated peptide coater on a streptavidin-coated microtitre plate and the supernatant from the growing monoclonal hybridoma. Specificities of the clones to a free peptide (HMGREGREGE), a non-sense peptide, and an elongated peptide (GHMGREGREG) were tested. Isotyping of the monoclonal antibodies was performed using the Clonotyping System-HRP kit, cat. no. 5300-05 (Southern Biotech, Birmingham, AL, USA). The selected clones were purified using protein G columns according to manufacturer's instructions (GE Healthcare Life Science, Little Chalfont, Buckinghamshire, UK). Selected monoclonal antibodies were labeled with horseradish peroxidase (HRP) using the Lightning link HRP labeling kit according to the instructions of the manufacturer (Innovabioscience, Babraham, Cambridge, UK).

### CO5-MMP ELISA methodology

In preliminary experiments, we optimized the reagents, their concentrations and the incubation periods by performing several checkerboard analyses. The CO5-MMP ELISA was developed as follows: A 96-well streptavidin plate was coated with 5 ng/mL biotinylated synthetic peptide HMGREGREGE-K-Biotin dissolved in assay buffer (25 mM Tris, 1% BSA, 0.1% Tween-20, pH 7.4) and incubated 30 minutes at 20°C by constant shaking at 300 rpm. Twenty µl of peptide calibrator or sample dissolved in assay buffer were added to appropriate wells, followed by 100 µL of conjugated monoclonal antibody (125 ng/mL) and incubated 1 hour at 4°C by constant shaking at 300 rpm. Finally, 100 µL tetramethylbenzinidine (TMB) (Kem-En-Tec cat. no. 438OH) was added and the plate was incubated 15 minutes at 20°C in the dark and shaking at 300 rpm. After each incubation step the plate was washed five times in washing buffer (20 mM Tris, 50 mM NaCl, pH 7.2). The TMB reaction was stopped by adding 100 µl stopping solution (1% HCL) and measured spectrophotometrically at 450 nm with 650 nm as the reference. A standard curve was performed by serial dilution of the CO5-MMP peptide (HMGREGREGE) and plotted using a 4-parametric mathematical fit model. Standard concentrations were 0, 15.625, 31.25, 62.5, 125, 250, 500, 1000 ng/mL.

### Technical evaluation

From 2-fold dilutions of pooled serum samples, linearity was calculated as a percentage of recovery of the 100% sample. The lower detection limit (LDL) was calculated from 21 determinations of the lowest standard (the zero standard) and calculated as the mean + 3x standard deviation. The inter- and intra-assay variation was determined by 10 independent runs of 5 QC samples, with each run consisting of two replicas of double determinations of the samples. Finally, for each assay, a master calibrator prepared from synthetic peptides accurately quantified by amino acid analysis was used for calibration purposes.

The analyte stability was determined for three human serum samples for 10 freeze and thaw cycles.

### Example 3: ELISA characterization

The developed CO5-MMP ELISA was evaluated using 20 µl of the samples: intact type V collagen, type V collagen cleaved with MMP-2, type V collagen cleaved with MMP-9, type V collagen cleaved with MMP-13, and an elongated CO5-MMP amino acid sequence (GHMGREGREG). Cross reactivity was tested using *in vitro* cleaved collagen type I.

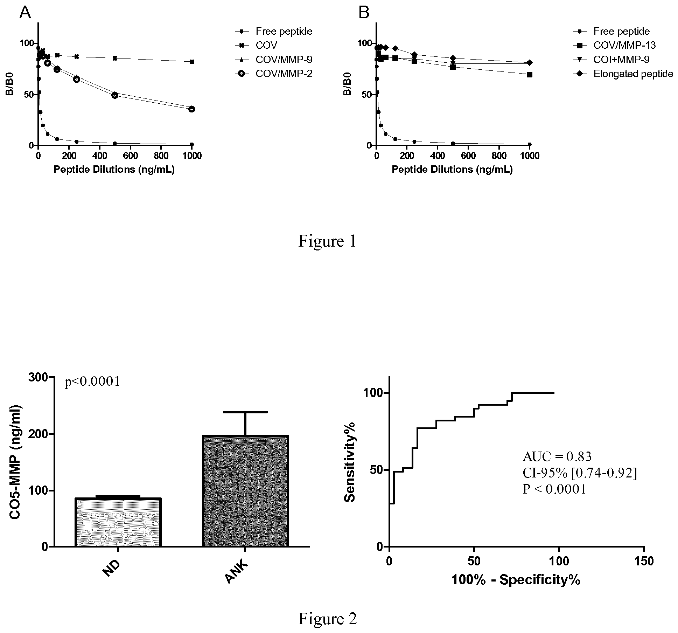

Results are shown in Figure 1, panels A and B, showing percent inhibition of the signal of: A) Free-peptide (HMGREGREGE), Intact type V collagen, MMP-9 cleaved type V collagen, MMP-2 cleaved type V collagen; B) Free-peptide (HMGREGREGE), MMP-13 cleaved type V collagen, MMP-9 cleaved type I collagn, elongated peptide (GHMGREGREG). It can be seen in panel A that the antibody is not substantially reactive with intact collagen type V but is reactive with cleaved collagen type V, and in panel B that the antibody is not reactive with the N-terminal extended peptide. The antibody is thus shown to be specifically reactive with the N-terminal neo-epitope produced by enzyme cleavage.

### Example 4: Clinical utility

### Healthy subjects and AS patients

The biochemical markers were assessed in serum from patients diagnosed with Ankylosing Spondylitis (AS, according to the modified New York criteria) and compared to sex and age matched non-diseased serum samples from the Department of Medicine 3 of the University of Erlangen-Nuremberg. The non-disease group consisted of 21 healthy females and 19 healthy males with a mean age of 43.0 years, range 18 to 66. The AS group consisted of 19 females and 21 males with a mean age of 42.5 years, range 29 to 63 years.

### Statistics

The serum levels of CO5-MMP between the two groups were measured using the ELISA (results shown in Figure 2) and compared using two-sided non-parametric Mann-Whitney test. In Figure 2, bars indicate mean levels ± standard error of the mean (SEM). Area under the curve was measured on ROC. Odds-ratios was extrapolated from contingency table were all subjects were classified as having low (within 2SD of the mean of the normal population) or high (>SD) levels of the biomarker. Results were considered significant when P<0.05.

These results strongly suggest that the measured neo-epitope of collagen Type V is a valuable marker for AS.

### Example 5 NB202 Biglycan assay: BGM .YWEVQPATFR SEQ ID NO 113

We demonstrate here the development of an enzyme-linked immunosorbent assay (ELISA) for the measurement of a MMP-9, MMP-12 and MMP-13-generated BGN neo-epitope and test the assay's biological validity in two rat models of liver fibrosis. **Methods:** BGN was cleaved *in vitro* by MMP-9, -12 and -13 and the ^{⇓344}'YWEVQPATFR'³⁵³ peptide (BGN-344) was chosen as a potential neo-epitope. A monoclonal mouse antibody raised against the selected peptide was used in the development of a competitive ELISA, measuring the MMP-degraded BGN neo-epitope. The assay was validated in two rat models of liver fibrosis, the carbon-tetrachloride (CCL₄)-induced fibrosis model, and the bile duct ligation (BDL) model.

**Results:** Significant elevation in serum BGN-344 was found in rats treated with CCL₄ for 16 weeks (200 ng/ml vs. 120 ng/ml, p=0.0013) and 20 weeks (190 ng/ml vs. 100 ng/ml, p=0.019) compared to the control groups as well as in all groups of rats subject to BDL compared with sham operated groups. Furthermore, there was a significant correlation of serum BGN-344 levels with the extent of liver fibrosis determined by the Sirius red staining of liver sections in the CCL₄ model.

**Conclusion:** We demonstrated the specific pathological tissue remodeling product of MMPs-degraded BGN, namely the neo-epitope BGN-344, is increased during liver fibrosis in a CCL₄ model and in a BDL model at both *in vivo* and *ex vivo* levels.

### Assay development:

The assay development of the selected monoclonal antibodies obtained from fusion included the following overall tests, according to the SOPs (data not shown): 1) Checkerboard (i.e. antibody-antigen binding affinity) - antibody vs. coater concentrations at different incubation time, incubation temperature and incubation buffer; 2) Displacement (i.e. competitive strength), to assess antibody specificity and sensitivity to a standard peptide, nonsense peptide, human serum, native BGN, native BGN *in vitro* processed with MMP-9, MMP-12 and MMP-13; 3) Labeling affinity to peroxidase, assessed using the Lightning-Link™ Horseradish Peroxidase labeling kit (Innova Bioscience Ltd, Babraham, UK). Based on the results of these tests the monoclonal antibody NB202-7-5A1 was selected for final assay development.

The BGN-344 competitive ELISA procedure was developed as follows: a 96-well streptavidin-coated plate was coated with 1.25 ng/ml YWEVQPATFR-K-Biotin (SEQ ID NO 305) for 30 min, at 20°C, at 300 rpm. The wells were then washed 5 times with standard washing buffer. A standard row was prepared by 2-fold pre-dilution of the standard peptide (**YWEVQP**ATFR) in 50 mM Tris-BTB starting from 250 ng/ml. Samples (i.e. human serum, mouse serum, rat serum) were likewise diluted. Standards and samples were added [20 µl/well], followed by the addition of 100 µl/well of 20 ng/ml peroxidase-labeled NB202-7-5A1 antibody in 50mM Tris BTB buffer. The plates were incubated for 1 hour at 4°C at 300 rpm. After the incubation period, the wells were washed 5 times and incubated with 100 µl of TMB at 4°C, 300 rpm, for 15 min, followed by the addition of 100 µl stop solution in each well. The colorimetric reaction was measured at 450 nm with reference at 650 nm on a standard laboratory plate reader. Data was acquired with the SoftMax Pro v5.0 program.

### Rat model of CCL₄ induced liver fibrosis:

BGN-344 levels were measured in a CCL₄ inhalation rat model of liver fibrosis. Complete details of the study are described elsewhere (133). The study included 52 male Wistar rats treated with CCL₄ and 28 male Wistar control rats (Charles-River, Saint Aubin les Elseuf, France). Induction of liver fibrosis was performed as previously described (134). Briefly, CCL₄ was administered by inhalation twice weekly and phenobarbital (0.3 g/l) added to the drinking water. Control rats received phenobarbital only. Animals were stratified into groups receiving 8, 12, 16 or 20 weeks of CCL₄ or control treatment (n=13 for CCL₄; n=7 control for each group). The study was performed after the specifically approved written informed consent by the Investigation and Ethics Committee of the Hospital Clinic Universitari (Barcelona, Spain), approval #B-NNP-233/09. Four animals from the CCL₄ groups died during the study. Blood was collected at termination and allowed to stand at room temperature for 20 min to allow clotting, before centrifugation at 2500 rpm for 10 min. Samples were stored at -80°C prior to biomarker assessment. Liver sections (4 µm thick) were stained in 0.1% Sirius red F3B (Sigma-Aldrich, St. Louis, MO) in saturated picric acid (Sigma-Aldrich). From each animal analyzed, the amount of fibrosis was expressed as a percentage of the total liver area of 36 fields and the average value of presented (135).

### Rat model of bile duct ligation (BDL) induced liver fibrosis:

BGN-344 levels were measured in a rat model of liver fibrosis induced by bile duct ligation. Complete details of the study are described elsewhere (133, 136). The study included a total of 81 female Sprague-Dawley rats aged 6 months. Liver fibrosis was induced in anaesthetized rats by standard BDL in which the bile duct was ligated in two places and dissected between the ligations prior to closing the abdomen. In sham-operated rats, the abdomen was closed without BDL.

The rats were divided into four groups: group 1 (10 BDL, 8 sham) were killed after one week, group 2 (12 BDL, 8 sham) killed after two weeks, group 3 (13 BDL, 8 sham) killed after three weeks, and group 4 (14 BDL, 8 sham) killed after four weeks.

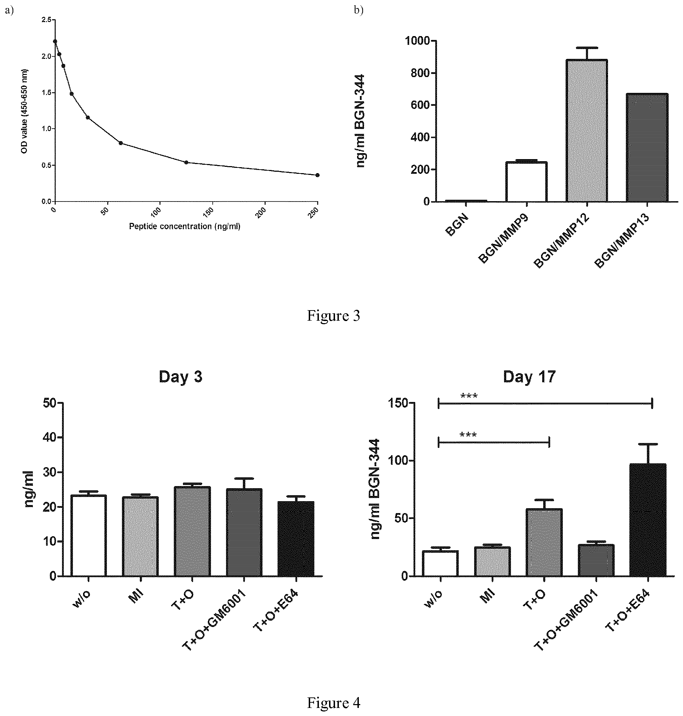

Figure 3 shows a) Example of the standard curve in a competitive ELISA setting b) BGN-344 peptide measurement for *in vitro* MMP-9, MMP-12 and MMP-13-degraded biglycan (BGN/MMP9, BGN/MMP12 and BGN/MMP13) and for intact biglycan (BGN).

Figure 4 shows BGN-344 peptide release from *ex vivo* cultures of bovine explants (BEX), measured at day 3 and 17 of culturing on five replicates. w/o=cultures without any stimulation; MI=metabolically inactive cultures by the treatment with liquid nitrogen; T+O= catabolic cytokine stimulation with TNFα [20 ng/ml] and oncostatin [10 ng/ml]; T+O+GM6001=catabolic cytokines TNF α [20 ng/ml] and oncostatin [10 ng/ml] supplemented with selective MMP inhibitor GM6001; T+O+E64= catabolic cytokines TNF α [20 ng/ml] and oncostatin [10 ng/ml] supplemented with selective cysteine protease inhibitor E64 (***=p<0.001).

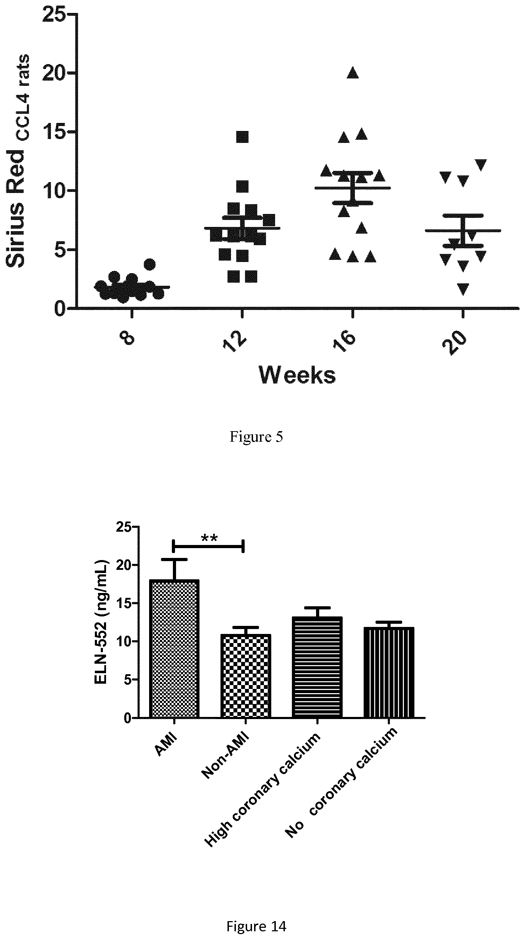

Figure 5 shows absolute values of Sirius red quantification in CCL4-treated rats determined at 8, 12, 16, and 20 weeks of treatment. (CCL₄ treated animals: n=13; controls: n=7 for each group).

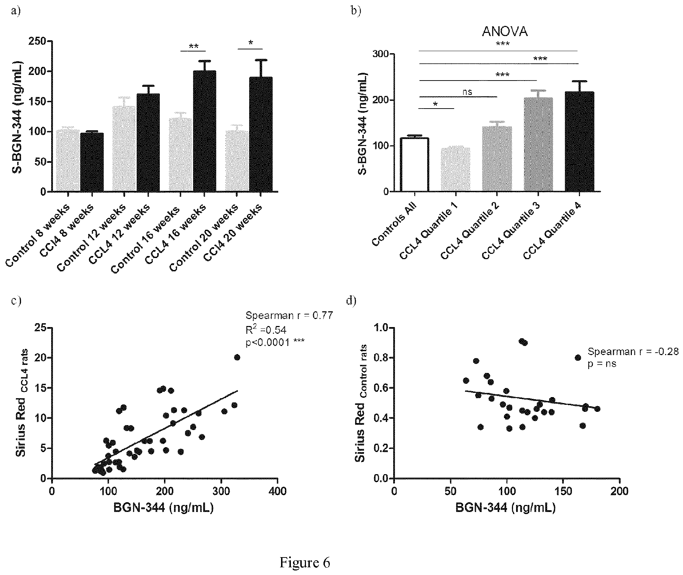

Figure 6 shows a) Mean Circulating serum BGN-344 levels in carbon tetra-chloride (CCL₄) induced rat model of liver fibrosis measured at 8, 12, 16, and 20 weeks (CCL₄ treated animals: n=13; controls: n=7 for each group). b) Serum BGN-344 levels in all control rats and all CCL₄ rat stratified in quartiles according to total collagen content in the liver determined by Sirius red; (ns=non significant, *=p<0.05, **=p<0.01, ***=p<0.001). Correlation of circulating serum BGN-344 and histological Sirius Red quantification determining the extent of liver fibrosis in c) CCL₄-treated rats and d) controls.

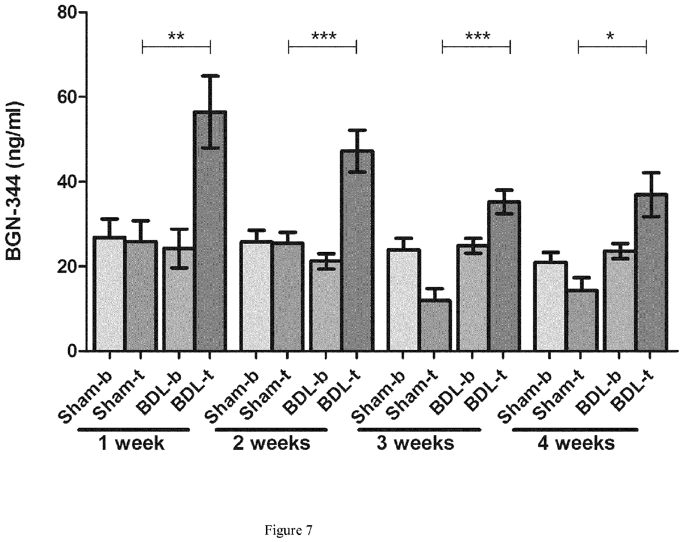

Figure 7 shows mean circulating serum BGN-344 levels in in BDL and sham operated rats at baseline and at termination at weeks 1, 2, 3, 4. Data are shown as mean ±standard error of the mean (SEM) (*=p<0.05, **=p<0.01, ***=p<0.001).

### Example 6: NB91 Type I collagen assay (FAP generated): .AGKEGPVGLP (SEQ ID NO 49) CO1-462 assay protocol

We labelled a selected monoclonal antibody with horseradish peroxidase (HRP) using the Lightning link HRP labeling kit according to the instructions of the manufacturer (Innovabioscience, Babraham, Cambridge, UK). A 96-well streptavidin plate was coated with biotinylated synthetic peptide AGKEGPVGLP-Biotin dissolved in assay buffer (50 mM Tris, 1% BSA, 0.1% Tween-20, pH 7.4 adjusted at 20°C) and incubated 30 minutes at 20°C. 20 µL of peptide calibrator or sample were added to appropriate wells, followed by 100 µL of conjugated monoclonal antibody 3H2-HRP and incubated 1 hour at 20°C. Finally, 100 µL tetramethylbenzinidine (TMB) (Kem-En-Tec cat.438OH) was added and the plate was incubated 15 minutes at 20oC in the dark. All the above incubation steps included shaking at 300 rpm. After each incubation step the plate was washed five times in washing buffer (20 mM Tris, 50 mM NaCl, pH 7.2). The TMB reaction was stopped by adding 100 µL of stopping solution (1% HCl) and measured at 450 nm with 650 nm as the reference. A calibration curve was plotted using a 4-parametric mathematical fit model.

### Rat bile duct ligation liver fibrosis model:

CO1-462 was assessed in a BDL rat model of liver fibrosis. The Danish animal welfare board "Dyreforsøgstilsynet" in Copenhagen, DK, had approved and given a written informed consent of the study; approval #2008/561-1450. The study has been described in detail elsewhere (Veidal et al. 2010b). Briefly, 17 female Sprague-Dawley rats aged 6 months were stratified into 4 groups: BDL- or sham-operated rats sacrificed after 2 or 4 weeks. Liver fibrosis was induced in anaesthetized rats by standard BDL, in which the bile duct was ligated in two places and dissected between the two ligations prior to closing the abdomen. In sham-operated rats, the abdomen was closed without BDL surgery. Blood samples were taken under CO₂/O₂ anaesthesia at baseline and at termination from the retro-orbital sinus of rats which had fasted for at least the previous 14 hours. The collected blood was left for 30 minutes at room temperature to clot, followed by centrifugation 2x at 1500g for 10 minutes. The serum was then transferred to clean tubes and stored at -80°C until use. The validity of the BDL model was demonstrated by (137).

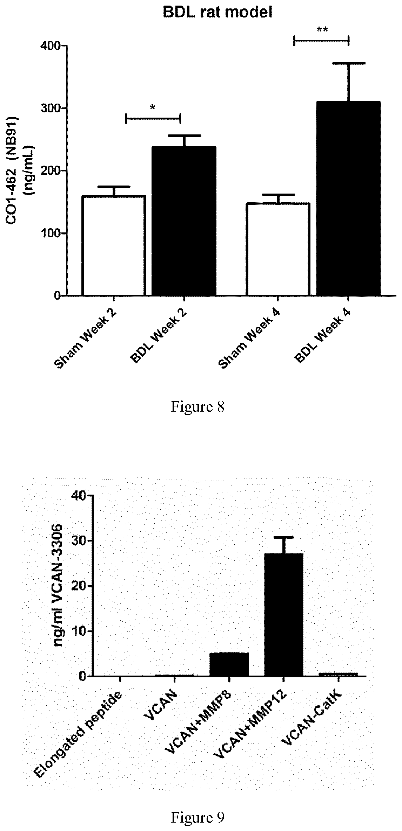

Results are shown in Figure 8. Significant elevation of the measured signal was seen after two weeks.

### Example 7 NB158 Versican assay: VCAN 3306 (.KTFGKMKPRY)

Extracellular matrix remodeling is a key feature in atherosclerotic lesion development and a prerequisite for plaque rupture. The proteoglycan versican is located in atherosclerotic plaques and it is a potential substrate for MMPs present in macrophage rich areas. We have developed an immunoassay to detect a specific MMP12 derived versican degradation fragment (VCAN-3306) and assess its potential as a biomarker for diagnosing atherosclerosis.

**Methods and Results:** A mouse monoclonal antibody raised against VCAN-3306 was used for the development of a competitive ELISA. Clinical plasma samples from patients with different degrees of atherosclerosis were used for the assay validation. The VCAN-3306 in coronary arteries of five deceased adults was detected using the VCAN-3306 mAb. Arteries with the lowest plaque burden displayed very little or no reactivity, compared to arteries affected by intermediate and large atherosclerotic plaques, which displayed high reactivity towards VCAN-3306 mAb. Patients experiencing I) acute coronary syndrome (n=30), II) stable ischemic heart disease (n=30) and III) demonstrating high levels of coronary calcium deposits (n=30) had significantly higher plasma levels of VCAN-3306 compared to a control group of individuals (n=30) with no detectable coronary calcium deposits. Moreover, a strong association was demonstrated between the presence of VCAN-3306 and LDL and total-cholesterol in patients with high levels of coronary calcium deposits.

**Conclusions:** Neoepitopes of versican generated by MMP12 are present in human atherosclerotic plaques. MMP12 mediated versican degradation was significantly elevated in patients with atherosclerotic diseases, suggesting VCAN-3306 as a potential biomarker for atherosclerosis.

### VCAN-3306 assay protocol:

The selected monoclonal antibodies were labeled with horseradish peroxidase (HRP) using the Lightning-Link Horseradish Peroxidase (HRP) antibody labeling kit according to the manufacturer's instructions (Innova Bioscience, Babraham, Cambridge, UK). A 96-well streptavidin pre-coated plate purchased from Roche Diagnostics (Roche Diagnostics, Basel, Switzerland) was coated with 2.5ng of the biotinylated synthetic peptide, KTFGKMKPRY-K-Biotin (SEQ ID NO 306), dissolved in assay buffer 50mM PBS BTB and incubated for 30 minutes at 20°C. 20µL of the peptide calibrator or sample were added to appropriate wells, followed by 100 µL of 20 ng/ml conjugated monoclonal antibody, and incubated for 1 hour at 20°C. Finally, 100 µL tetramethyl benzinidine (TMB) (Kem-En-Tec cat.438OH, Taastrup, Denmark) developer was added, and the plate was incubated for 15 minutes at 20°C in the dark. All the above incubation steps included shaking at 300 rpm. After each incubation step the plate was washed five times in washing buffer (20mM Tris, 50mM NaCl, pH 7.2). The TMB reaction was stopped by adding 100 µL of stopping solution (1% HCl) and measured at 450nm with 650nm as the reference. A standard calibration curve was plotted using a 4-parametric mathematical fit model with a starting concentration of 10 ng for the standard peptide following a 2-fold dilution and the last standard was a zero standard.

### Samples and procedures for immunohistochemistry:

Specimens: Coronary arteries were obtained from patients older than 25 years who died during an 18-month period at North Carolina Baptist Hospital, Winston-Salem, USA, as previously described (138).

Histopathologic procedures: Five hearts obtained at autopsy were flushed with normal saline and were perfusion fixed with 4% neutral buffered formaldehyde solution at a pressure of 100 mm Hg for 1 hour. Adjacent tissue blocks for histological sectioning (each 3 mm long) were cut perpendicular to the long axis (proximal 3 cm) of the left anterior descending (LAD), the left circumflex, and the right coronary arteries. Two 5-µm histological sections were made from each block. One was stained with hematoxylin-eosin and one with Verhoeff-van Gieson stain.

The standard Nordic Bioscience protocol was used for VCAN-3306 staining of human left anterior descending coronary arteries from the five individuals, each with a different degree of atherosclerosis. Briefly, the paraffin embedded arteries were cut into 5 µm sections, de-paraffinized, and peroxidase blocked and rehydrated. The sections were subjected to pre-treatment 2x5 min in a microwave at 800W. Hereafter, the sections were washed in TBC+1%Triton X-100, blocked in 0.5% casein in TBS and incubated with 2.25µg/ml mAb against VCAN-3306 diluted in TBS buffer over night at 4°C. The sections were subsequently washed in TBS+1% Triton X-100, incubated in Super Sensitive for 30 min, washed again and treated with DAB substrate (6-10 min). The sections were immersed in Mayers Hematoxylin for 12 seconds, mounted and left to dry.

### Patients and sample collection:

Four groups of 30 individuals each, with different degrees of atherosclerotic heart disease, were selected from three large studies (DANRISK, DEFAMI and Odense Artery Biobank). Participants in these studies were enrolled simultaneously by Odense University Hospital, Odense, Denmark. The four groups consisted of: 1) 30 asymptomatic individuals with no detectable coronary calcium (CT-noCa), 2) 30 asymptomatic subjects with subclinical CVD defined by the presence of coronary calcium (CT-plusCa), 3) 30 patients with stable ischemic heart disease (IHD), bound for coronary by-pass surgery and 4) 30 patients with acute coronary syndrome (ACS) and symptom onset within the previous 24 hours (Table1). All samples were handled and tested by the Department of Clinical Biochemistry and Pharmacology at Odense University Hospital (Odense, Denmark).

The group 1 and 2 patients were recruited from the DANRISK-cohort, a random sample of 1825 men and women aged 50 to 60 years old, who in 2009 were invited for screening for coronary disease. The screening included a cardiac CT-scan, followed by an estimation of the amount of coronary calcium using the Agatston score. From the invited individuals, 1257 were considered for screening, but after exclusion of those with prior ischemic heart disease or diabetes mellitus (n=100), 1157 patients were enrolled for analysis. Among these individuals aged 60 years (n=647), 30 patients without detectable coronary calcium (CT-noCa), group 1, and 30 with an Agatston score ≥400 (CT-plusCa) group 2, were chosen. The selection of these two groups also involved matching for total cholesterol and blood pressure. HeartScore is aimed at supporting clinicians in optimizing individual cardiovascular risk reduction. This risk estimation is based on the following risk factors: gender, age, smoking, systolic blood pressure and total cholesterol, and it will predict the number of fatal CVD-events that eventually will occur after 10 years. The threshold for high risk based on fatal cardiovascular events is defined as "higher than 5%" (139).

The group of patients with stable IHD, group 3, came from The Odense Artery Biobank, which gathers spare arterial tissue and blood samples from patients undergoing coronary bypass surgery. Blood samples were taken the day before the by-pass operation. We selected 30 patients with a mean age of approximately 60 years and a gender distribution similar to the above-mentioned populations. The group of patients with ACS, group 4, is recruited from the DEFAMI-cohort, in which all patients were admitted to Odense University Hospital between October 1 2009 and April 30 2010 and had troponin analysis performed on suspicion of ACS were enrolled. In these patients, serial blood sampling was performed within the first 24 hours of symptom onset. Of the 822 patients in the DEFAMI cohort, 30 patients with non-ST elevation myocardial infarction (NSTEMI, n=24) or unstable angina pectoris (n=6) were selected to participate in the current study. Their age was approximately 60 years and their gender distribution matched the two DANRISK subgroups (group 1 and group 2). NSTEMI was defined if a typical rise and/or fall of TnI levels above 0.03 µg/l was demonstrated in association with the presence of typical clinical symptoms. Unstable angina pectoris was defined as the presence of typical chest pain during rest or minimal physical exertion together with ST-segment depression or T-wave fluctuations, but with normal TnI levels (below 0.03 pg/l). All patients with unstable angina pectoris also had to demonstrate at least one significant coronary artery stenosis (> 50% diameter stenosis). Thus, in this study ACS patients included patients with NSTEMI and unstable angina pectoris. Patients with ST-segment elevation myocardial infarction (STEMI) were not included, because in these patients it would not be possible to obtain sufficient serial blood samples before medication with heparin and acute PCI were done. Both interventions might interfere in biochemical assays. The mean TnI level in the 30 ACS patients (group 4) was 0.62 pg/l (range 0.01-5.23 µg/l).

In all patients, group1-4 (n=120), hypertension was considered if the patient used antihypertensive medical treatment and diabetes was defined if anti-diabetic medication was prescribed. Systolic and diastolic blood pressure was measured on the same day as blood sampling. Agatston score was calculated in group 1 and 2 patients. TnI in the ACS group was measured immediately on hospital admittance, prior to the current blood sampling. Total cholesterol, LDL, HDL and triglycerides were also measured prior to blood sampling for the present study. Blood samples were drawn in tubes with EDTA and centrifuged at 200g for 10 min. Plasma was stored at -80°C until biochemical analysis.

The study protocols for the DANRISK, DEFAMI and Odense Artery Biobank studies were approved by the Regional Ethics Committee before the initiation of the studies. Informed consent was obtained from all participating patients.

**Table 50. Demographics of each group included in the study with mean values and standard deviation in [].**

| | **CT-noCa n=30** | **CT-plus Ca n=30** | **ACS n=30** | **IHD n=30** | **ANOVA p-value** |
|---|---|---|---|---|---|
| **Age (years)** | 60.2 [59.8; 60.7] | 60.3 [60.0-60.6] | 64.5** [56.0; 73.0] | 59.7 [56.8; 62.6] | 0.0001 |
| **Triglycerides^{#} (mmol/L)** | 1.57 [0.85; 2.93] | 1.49 [1.00; 2.21] | 1.30 [0.78; 2.19] | 1.48 [0.92; 2.30] | 0.52 |
| **HDL Cholesterol^{#} (mmol/L)** | 1.28 [0.93; 1.75] | 1.36 [0.98; 1.89] | 1.13 [0.83; 1.52] | 1.30 [0.90;1.87] | 0.14 |
| **LDL Cholesterol (mmol/L)** | 3.14 [2.13; 4.16] | 3.26 [2.29; 4.22] | 2.93 [1.74; 4.11] | 2.41* [1.27; 3.55] | 0.02 |
| **Total Cholesterol (mmol/L)** | 5.25 [4.06; 6.43] | 5.47 [4.29; 6.64] | 4.74 [3.44; 6.04] | 4.45* [3.20; 5.71] | 0.007 |
| **Systolic Blood Pressure (mmHg)** | 145 [131; 159] | 147 [128; 167] | 159* [132; 187] | 145 [123; 168] | 0.04 |
| **Diastolic Blood Pressure (mmHg)** | 86 [76; 95] | 85 [75; 96] | 88 [72; 103] | 83 [74; 92] | 0.56 |
| **Agatston score^{#}** | 0 | 1299 [717; 2352] | - | - | - |
| **Heart Score^{#}** | 6.33 [2.23; 8.92] | 8.30 [3.10;12.42] | - | - | 0.3 |

| | | | | | |
|---|---|---|---|---|---|
| CT-noCa: Asymptomatic individuals without detectable coronary calcium; CT-plusCa: Asymptomatic CVD with high coronary calcium; ACS (Acute Coronary Syndrome); IHD: patients with stable ischemic heart disease. Mean values [+/- STD)]; # Geometric mean values [+/- STD)], and p-value from one-way of analysis of variance (ANOVA). The level of significance of p-values from comparison of each group against the control group 'CT-noCa' was adjusted for multiple comparisons by the method of Dunnet (*: p<0.05; **: p<0.01; ***: p<0.001). | | | | | |

**Table 51. The results of the ROC curves in high coronary calcium-(CT-plus Ca), acute coronary syndrome-(ACS) and ischemic heart disease-(IHD) group compared to the control group with no detectable coronary calcium (CT-no Ca). AUC is given with standard error and p-values.**

| **ROC curve; AUC [std error] p-value** | | | |
|---|---|---|---|
| **Parameter** | **CT-plus Ca vs CT-no Ca** | **ACS vs CT-no Ca** | **IHD vs CT-no Ca** |
| **VCAN-3306** | 0.748 | 0.775 | 0.669 |
| | [0.068] | [0.062] | [0.077] |
| | 0.0003 | <0.0001 | 0.03 |

**Table 52. Spearman correlation coefficients of VCAN-3306 and standard measured parameters are presented with r-values and p-values.**

| **Spearman Correlation Coefficients** | **All groups r-value p-value** | **CT-plus Ca** | **ACS** | **IHD** |
|---|---|---|---|---|
| **Age** | 0.1675 | -0.0147 | - | 0.0769 |
| | 0.4038 | 0.9386 | 0.1387 | 0.7027 |
| | | | 0.4647 | |
| **Triglycerides** | -0.1011 | 0.3281 | 0.1409 | -0.0806 |
| | 0.6160 | 0.0767 | 0.4577 | 0.6895 |
| **LDL** | -0.2291 | 0.4665 | 0.3186 | -0.1394 |
| | 0.2503 | **0.0094**** | 0.0862 | 0.4880 |
| **HDL** | -0.0501 | 0.1945 | 0.0309 | 0.2688 |
| | 0.8041 | 0.3030 | 0.8714 | 0.1752 |
| **Total** | -0.2062 | 0.5993 | 0.3187 | 0.0504 |
| **Cholesterol** | 0.3022 | **0.0005***** | 0.0860 | 0.8028 |
| **BP-Systolic** | 0.2745 | 0.0982 | 0.1953 | -0.0527 |
| | 0.1659 | 0.6057 | 0.3011 | 0.8304 |
| **BP-Diastolic** | 0.0266 | 0.2060 | 0.0720 | 0.0330 |
| | 0.8952 | 0.2747 | 0.7053 | 0.8933 |

Figure 9 shows the results of a test of antibody specificity towards its epitope. Samples added and tested in a competitive ELISA setting were: elongated peptide (20 ng/ml), uncleaved versican (VCAN), MMP-8 cleaved versican (VCAN+MMP8), MMP-12 cleaved versican (VCAN+MMP12) and Cathepsin K cleaved versican (VCAN+CatK).

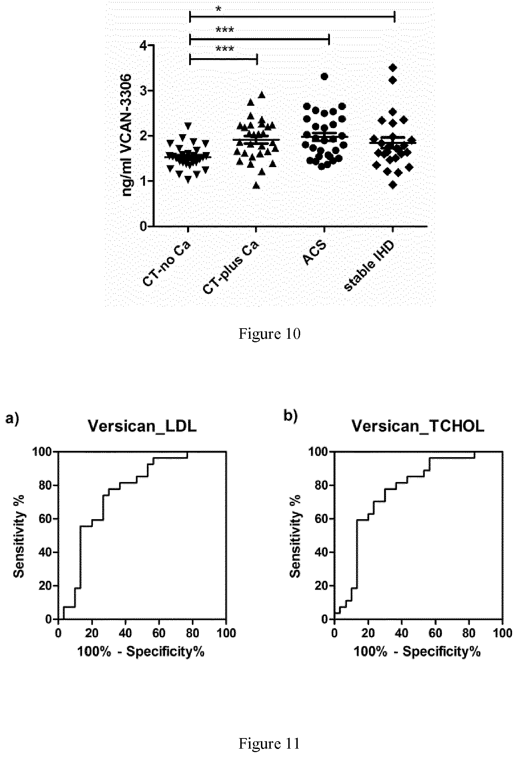

Figure 10 shows plasma levels of VCAN-3306 measured in patients diagnosed with acute coronary syndrome (ACS), high deposits of coronary calcium (CT-plus Ca), stable ischemic heart disease (IHD) and compared with age-matched controls comprising individuals with no detectable coronary calcium deposits (CT-no Ca). * = p<0.05; *** = p<0.0005; Shown are individual values and the group mean and SEM as line and error bars.

Figure 11 shows ROC curves for a) VCAN-3306 combined with LDL and b) VCAN-3306 combined with cholesterol in the group of asymptomatic patients with high coronary calcium deposits (CT-plus Ca) .

### Example 8 NB185 Type V assay: C5M .HMGREGREGE (SEQ ID NO 93)

The chronic inflammation Ankylosing Spondylitis (AS) of the spine can lead to a complete cementation of the vertebrae, a process that include turnover of several collagens found in the joint. Diagnostic assessment and patient characterization may be improved by measurement of connective tissue turnover markers. Proteolytic degradation of collagens plays an important role in extracellular matrix remodeling and thereby in the pathogenesis of AS. This degradation creates small peptide fragments called neo-epitopes, which may prove to be a new class of biomarkers of fibrotic events. The aims of this study were to develop a competitive ELISA capable of detecting a fragment of type V collagen generated by MMP-2 and MMP-9 and to evaluate the use of this assay as biomarker for AS.

*Materials and methods*: Mass spectrometric analysis of type V collagen degraded by MMP-2 and MMP-9 revealed a large number of protease-generated neo-epitopes. From these data a fragment unique to type V collagen and generated by MMP-2 and MMP-9 was selected as a target for ELISA development. A monoclonal antibody was raised against this neo-epitope, which detected the type V collagen fragment at the amino acid position 1317 (CO5-MMP). The assay was used to assess the content of CO5-MMP neo-epitope in serum of 40 AS patients and 40 age-matched controls.

*Results*: The ELISA was demonstrated to detect the CO5-MMP neo-epitope fragment of MMP-2 and MMP-9 degradation exclusively with inter- and intra-assay variations of 9.13% and 4.38% respectively. The level of CO5-MMP was significantly higher in AS patients with an increase of 229% (P<0.0001). AUC was 83%.

*Conclusions*: This novel ELISA is the first assay developed for assessing type V collagen neo-epitopes. The high turnover of type V collagen suggests a pathophysiologic role. CO5-MMP is a candidate marker of ongoing fibrogenesis.

### Methods

### Healthy subjects and AS patients

The biochemical markers were assessed in serum from patients diagnosed with Ankylosing Spondylitis (AS, according to the modified New York criteria) and compared to sex and age matched non-diseased serum samples from the Department of Medicine 3 of the University of Erlangen-Nuremberg. The non-disease group consisted of 21 healthy females and 19 healthy males with a mean age of 43.0 years, range 18 to 66. The AS group consisted of 19 females and 21 males with a mean age of 42.5 years, range 29 to 63 years. (Table 53)

### CO5-MMP ELISA methodology

In preliminary experiments, we optimized the reagents, their concentrations and the incubation periods by performing several checkerboard analyses. The CO5-MMP ELISA was developed as follows: A 96-well streptavidin plate was coated with 5 ng/mL biotinylated synthetic peptide HMGREGREGE-K-Biotin (SEQ ID NO 307) dissolved in assay buffer (25 mM Tris, 1% BSA, 0.1% Tween-20, pH 7.4) and incubated 30 minutes at 20°C by constant shaking at 300 rpm. Twenty µl of peptide calibrator or sample dissolved in assay buffer were added to appropriate wells, followed by 100 µL of conjugated monoclonal antibody (125 ng/mL) and incubated 1 hour at 4°C by constant shaking at 300 rpm. Finally, 100 µL tetramethylbenzinidine (TMB) (Kem-En-Tec cat. no. 438OH) was added and the plate was incubated 15 minutes at 20°C in the dark and shaking at 300 rpm. After each incubation step the plate was washed five times in washing buffer (20 mM Tris, 50 mM NaCl, pH 7.2). The TMB reaction was stopped by adding 100 µl stopping solution (1% HCL) and measured spectrophotometrically at 450 nm with 650 nm as the reference. A standard curve was performed by serial dilution of the CO5-MMP peptide (HMGREGREGE) and plotted using a 4-parametric mathematical fit model. Standard concentrations were 0, 15.625, 31.25, 62.5, 125, 250, 500, 1000 ng/mL.

**Table 53. Description of the study**

| | Controls | AS |
|---|---|---|
| **N (Women/Men)** | 40 (21/19) | 40 (19/21) |
| **Age (range)** | 43.0 years (18-66) | 42.5 years (29-63) |
| **CRP [95% CI]** | 3016 ng/ml [1595, 4438] | 4061 ng/ml [2426, 5696] |
| **BASDAI** | | 3.79 [0.01, 0.45] |
| **mSASSS** | | 5.08 [0.04, 1.14] |

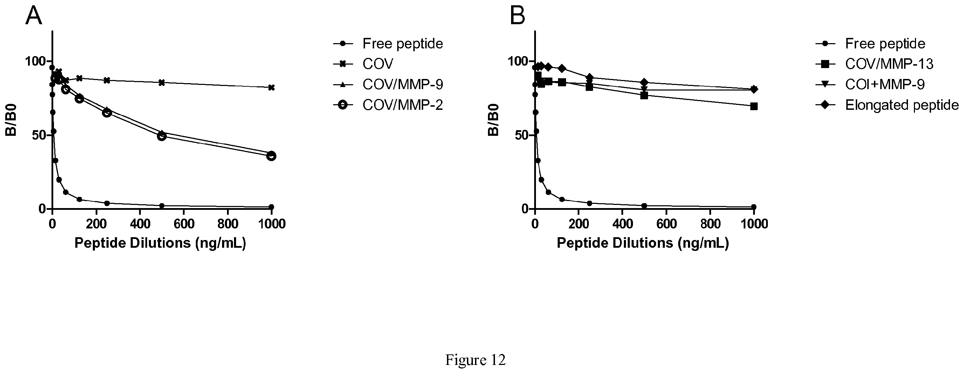

Figure 12 shows the results obtained in a characterization of the CO5-MMP ELISA showing percent inhibition of the signal of: A) Free-peptide (HMGREGREGE), Intact type V collagen, MMP-9 cleaved type V collagen, MMP-2 cleaved type V collagen; B) Free-peptide (HMGREGREGE), MMP-13 cleaved type V collagen, MMP-9 cleaved type I collagen, elongated peptide (GHMGREGREG).

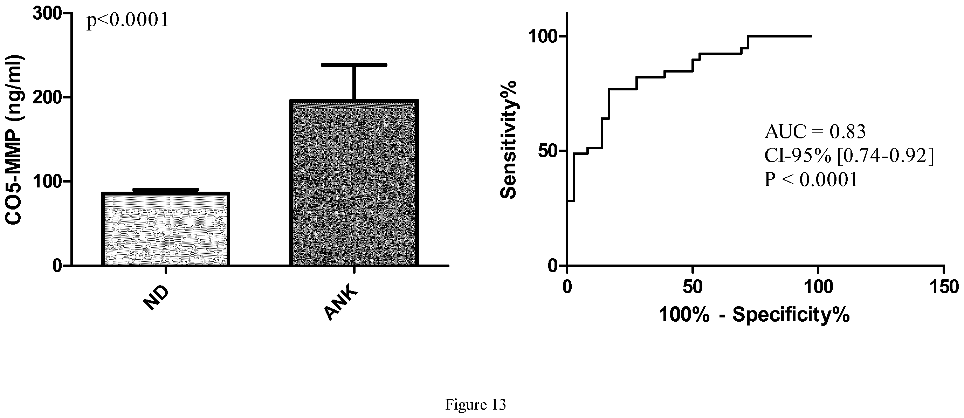

Figure 13 shows the results of **m**easurement of CO5-MMP in non diseased subjects (ND, n=40) and in Ankylosing Spondylitis patients (AS, n=40). Bars indicate mean levels ± standard error of the mean (SEM). Groups were compared by Mann-Whitney.

### Example 9 - Elastin ELN-552 (ELM-2) GVAPGIGPGG. (SEQ ID NO 308)

### Materials and Methods

**Table 54. Description of the elastin neo-epitope program. An arrow pointing down indicates the cleavage site and the elongating amino acid for the elongated peptide is underlined.**

| Program Number | Target sequence (human) | Description of target | Homology | ID | Selection and deselection peptides |
|---|---|---|---|---|---|
| NB244 | GIGPGG↓ Aa 547-552 | Elastin cleaved by MMP9/12 as a degradation marker | Human | *In vitro* and *in vivo* cleavages | gvapGIGPGG |
| | ELN-552 assay | | | | gvapGIGPGGVA SEQ ID NO 309 |

### ELISA procedure ELN-552 (ELM2)

Assay buffer and plates were equilibrated to room temperature. 100µL biotinylated peptide (biotin-CGG-GVAPGIGPGG SEQ ID NO 287) diluted to 2.5ng/mL in assay buffer (50mM PBS, 1% BSA, 0.1% Tween-20, pH 7.40) was used to coat 96-well streptavidin coated plates. Plates were incubated for 30 minutes at 20°C, shaking at 300 rpm and washed five times in washing buffer (20mM tris, 50mM NaCl, pH 7.2). Each serum sample was diluted 2-fold in assay buffer and 20µL of serum dilution or peptide calibrator (starting at 250ng/mL) was added to each well. 100µL HRP-labelled NB244-2B4 antibody at a concentration of 65ng/mL was added to each well and the plate was left to incubate for 1 hour at 20°C, shaking at 300 rpm. Plates were washed five times in washing buffer, 100µL TMB was added, and the plate was incubated 15 minutes at 20°C in darkness. 100µL of stopping solution (1% H₂SO₄) was added and the plate read in an ELISA reader at 450 nm with 650 nm as reference. A calibration curve was fitted using a 4-parameter mathematical fit model.

### Clinical Validation of the ELN-552 Assay

The ELN-552 assay was tested in the BioCore cohort, which is an atherosclerosis cohort. The cohort was run undiluted in the assay. Patients in the BioCore cohort are divided into four groups based on clinical symptoms: the AMI group, which is patients with confirmed acute myocardial infarction, the non-AMI group, which is patients with suspected AMI, but no diagnosis based on troponin-T levels, the high coronary calcium group, which are patients that present with high coronary calcium based on agatstonscore but no clinical symptoms, and the no coronary calcium group, which is the negative control group. Patients in the no coronary calcium group were randomly chosen based on their CPR number and have no clinical symptoms or biomarker indication of atherosclerosis. All are sex- and age-matched to the rest of the cohort. In each group are 30 patients, making it a study of 120 patients. A demographic table of some of the BioCore data is seen in Table 55.

**Table 55. The demographical data over the BioCore cohort.**

| *Group* | *Age (years)* | *Sys. BP (mmHg)* | *Dias. BP (mmHg)* | *Total cholesterol (mmol*/*L)* | *HDL (mmol*/*L)* | *LDL (mmol*/*L)* | *Agatston Score* | *Heart score** |
|---|---|---|---|---|---|---|---|---|
| AMI (n=30) | 64.5±9 | 159±28 | 88±16 | 4.7±1.3 | 1.2±0.3 | 2.9±1.2 | --- | 9.7±5.4 |
| Non-AMI (n=30) | 60.2±8 | 143±26 | 81±14 | 4.9±0.9 | 1.5±0.7 | 2.7±0.7 | 498** | 8.7±5.7 |
| High Cor Ca (n=30) | 60.3±0.3 | 147±20 | 86±11 | 5.5±1.2 | 1.4±0.5 | 3.3±1 | 1509±1070 | 8.3±6.2 |
| No Cor Ca (n=30) | 60.3±0.4 | 145±14 | 86±10 | 5.2±1.2 | 1.3±0.4 | 3.1±1 | 0.00 | 6.3±4.5 |

| | | | | | | | | |
|---|---|---|---|---|---|---|---|---|
| Values are mean ± standard deviation. High Cor Ca: the high coronary calcium group, No Cor Ca: the no coronary calcium group. BP: blood pressure *Heart score is an assessment on risk of cardiovascular disease based on age, systolic blood pressure, total cholesterol in mmol/L, and smoking status. **Agatston score from only one patient. | | | | | | | | |

### Statistics

Upon testing the two cohorts in the ELN-552 assay the data was checked for Gaussian distribution using a D'Agostino-Pearson omnibus test and visually checking distribution by creating a histogram. The different groups were then tested for difference using a non-parametric t-test.

### Results

### ELN-552 is elevated in patients with Acute Myocardial Infarction

The ELN-552 was tested in the BioCore atherosclerosis cohort. Levels of ELN-552 were significantly higher in serum from patients with AMI compared to non-AMI (p<0.01). Patients are divided into groups based on clinical symptoms and imaging markers. The bar covering the AMI and non-AMI groups indicates a significant difference between these groups calculated by a non-parametric t-test.** indicates p-value less than 0.01. For comparison the y-axis has the same value as in. Using a non-parametric t-test it is evident that the highest difference between groups is found between the AMI and non-AMI groups. Between the AMI and no coronary calcium group there was an almost significant difference of p=0.07 and between the non-AMI and high coronary calcium groups there was a p-value of 0.08, suggesting that larger patient groups and thereby more power could confirm these differences and further characterise the assay.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. No acknowledgement of any prior published document herein should be taken to be an admission or representation that the teaching thereof was common general knowledge in Australia or elsewhere at the date hereof.

### Reference List

1. World Health Organization. Reducing Risks, Promoting Healthy Life. Peducing Risks, Promoting Healthy Life, Geneva: WHO, 2002:1-230.
2. Wynn TA. Cellular and molecular mechanisms of fibrosis. J Pathol 2008;214:199-210.
3. Friedman SL. Mechanisms of disease: Mechanisms of hepatic fibrosis and therapeutic implications. Nat Clin Pract Gastroenterol Hepatol 2004;1:98-105.
4. Tomasek JJ, Gabbiani G, Hinz B, Chaponnier C, Brown RA. Myofibroblasts and mechano-regulation of connective tissue remodelling. Nat Rev Mol Cell Biol 2002;3:349-363.
5. Wynn TA. Common and unique mechanisms regulate fibrosis in various fibroproliferative diseases. J Clin Invest 2007;117:524-529.
6. Marcellin P, Asselah T, Boyer N. Fibrosis and disease progression in hepatitis C. Hepatology 2002;36:S47-S56.
7. Gagliano N, Arosio B, Grizzi F, Masson S, Tagliabue J, Dioguardi N, Vergani C, Annoni G. Reduced collagenolytic activity of matrix metalloproteinases and development of liver fibrosis in the aging rat. Mech Ageing Dev 2002;123:413-425.
8. Laurent GJ. Dynamic state of collagen: pathways of collagen degradation in vivo and their possible role in regulation of collagen mass. Am J Physiol 1987;252:C1-C9.
9. Mays PK, McAnulty RJ, Campa JS, Laurent GJ. Age-related changes in collagen synthesis and degradation in rat tissues. Importance of degradation of newly synthesized collagen in regulating collagen production. Biochem J 1991;276 (Pt 2):307-313.
10. Garrone R, Lethias C, Le Guellec D. Distribution of minor collagens during skin development. Microsc Res Tech 1997;38:407-412.
11. Gelse K, Poschl E, Aigner T. Collagens--structure, function, and biosynthesis. Adv Drug Deliv Rev 2003;55:1531-1546.
12. Phan SH, Thrall RS. Pulmonary Fibrosis. Lung Biology in Health and Disease. 80 ed. New York: Marcel Dekker, Inc., 1995.
13. Martinez-Hernandez A, Amenta PS. The hepatic extracellular matrix. II. Ontogenesis, regeneration and cirrhosis. Virchows Arch A Pathol Anat Histopathol 1993;423:77-84.
14. Gilliam AC. Scleroderma. Curr Dir Autoimmun 2008;10:258-279.
15. Gressner AM, Weiskirchen R. Modern pathogenetic concepts of liver fibrosis suggest stellate cells and TGF-beta as major players and therapeutic targets. J Cell Mol Med 2006;10:76-99.
16. Heinegard D, Oldberg A. Structure and biology of cartilage and bone matrix noncollagenous macromolecules. FASEB J 1989;3:2042-2051.
17. Svensson L, Oldberg A, Heinegard D. Collagen binding proteins. Osteoarthritis and Cartilage 2001;9:S23-S28.
18. Kiani C, Chen L, Wu YJ, Yee AJ, Yang BB. Structure and function of aggrecan. Cell Res 2002;12:19-32.
19. Krusius T, Gehlsen KR, Ruoslahti E. A fibroblast chondroitin sulfate proteoglycan core protein contains lectin-like and growth factor-like sequences. J Biol Chem 1987;262:13120-13125.
20. Yang BL, Zhang Y, Cao L, Yang BB. Cell adhesion and proliferation mediated through the G1 domain of versican. J Cell Biochem 1999;72:210-220.
21. Rauch U, Karthikeyan L, Maurel P, Margolis RU, Margolis RK. Cloning and primary structure of neurocan, a developmentally regulated, aggregating chondroitin sulfate proteoglycan of brain. J Biol Chem 1992;267:19536-19547.
22. Yamada H, Watanabe K, Shimonaka M, Yamaguchi Y. Molecular cloning of brevican, a novel brain proteoglycan of the aggrecan/versican family. J Biol Chem 1994;269:10119-10126.
23. Blochberger TC, Cornuet PK, Hassell JR. Isolation and partial characterization of lumican and decorin from adult chicken corneas. A keratan sulfate-containing isoform of decorin is developmentally regulated. J Biol Chem 1992;267:20613-20619.
24. Fisher LW, Termine JD, Young MF. Deduced protein sequence of bone small proteoglycan I (biglycan) shows homology with proteoglycan II (decorin) and several nonconnective tissue proteins in a variety of species. J Biol Chem 1989;264:4571-4576.
25. Toyama-Sorimachi N, Sorimachi H, Tobita Y, Kitamura F, Yagita H, Suzuki K, Miyasaka M. A novel ligand for CD44 is serglycin, a hematopoietic cell lineage-specific proteoglycan. Possible involvement in lymphoid cell adherence and activation. J Biol Chem 1995;270:7437-7444.
26. Bartlett AH, Hayashida K, Park PW. Molecular and cellular mechanisms of syndecans in tissue injury and inflammation. Mol Cells 2007;24:153-166.
27. Lopez-Casillas F, Wrana JL, Massague J. Betaglycan presents ligand to the TGF beta signaling receptor. Cell 1993;73:1435-1444.
28. Olsen BR. Life without perlecan has its problems. J Cell Biol 1999;147:909-912.
29. Gabay C, Kushner I. Acute-phase proteins and other systemic responses to inflammation. N Engl J Med 1999;340:448-454.
30. Benyon RC, Arthur MJ. Extracellular matrix degradation and the role of hepatic stellate cells. Semin Liver Dis 2001;21:373-384.
31. Guo J, Friedman SL. Hepatic fibrogenesis. Semin Liver Dis 2007;27:413-426.
32. Iredale JP, Benyon RC, Arthur MJ, Ferris WF, Alcolado R, Winwood PJ, Clark N, Murphy G. Tissue inhibitor of metalloproteinase-1 messenger RNA expression is enhanced relative to interstitial collagenase messenger RNA in experimental liver injury and fibrosis. Hepatology 1996;24:176-184.
33. Lee KN, Jackson KW, Christiansen VJ, Lee CS, Chun JG, McKee PA. Antiplasmin-cleaving enzyme is a soluble form of fibroblast activation protein. Blood 2006;107:1397-1404.
34. Acharya PS, Zukas A, Chandan V, Katzenstein AL, Pure E. Fibroblast activation protein: a serine protease expressed at the remodeling interface in idiopathic pulmonary fibrosis. Hum Pathol 2006;37:352-360.
35. Levy MT, McCaughan GW, Marinos G, Gorrell MD. Intrahepatic expression of the hepatic stellate cell marker fibroblast activation protein correlates with the degree of fibrosis in hepatitis C virus infection. Liver 2002;22:93-101.
36. Meyer O. Prognostic markers for systemic sclerosis. Joint Bone Spine 2006;73:490-494.
37. Hummers LK. Microvascular damage in systemic sclerosis: detection and monitoring with biomarkers. Curr Rheumatol Rep 2006;8:131-137.
38. McHugh NJ, Distler O, Giacomelli R, Riemekasten G. Non organ based laboratory markers in systemic sclerosis. Clin Exp Rheumatol 2003;21:S32-S38.
39. Muller-Quernheim J. Serum markers for the staging of disease activity of sarcoidosis and other interstitial lung diseases of unknown etiology. Sarcoidosis Vasc Diffuse Lung Dis 1998;15:22-37.
40. Gressner OA, Weiskirchen R, Gressner AM. Biomarkers of liver fibrosis: clinical translation of molecular pathogenesis or based on liver-dependent malfunction tests. Clin Chim Acta 2007;381:107-113.
41. Gressner OA, Weiskirchen R, Gressner AM. Biomarkers of hepatic fibrosis, fibrogenesis and genetic pre-disposition pending between fiction and reality. J Cell Mol Med 2007;11:1031-1051.
42. Mariat C. [Diagnosis and follow-up of chronic kidney graft dysfunction: from DFG to new biomarkers]. Nephrol Ther 2008;4 Suppl 3:S204-S207.
43. Yoneda M, Mawatari H, Fujita K, Iida H, Yonemitsu K, Kato S, Takahashi H, Kirikoshi H, Inamori M, Nozaki Y, Abe Y, Kubota K, Saito S, Iwasaki T, Terauchi Y, Togo S, Maeyama S, Nakajima A. High-sensitivity C-reactive protein is an independent clinical feature of nonalcoholic steatohepatitis (NASH) and also of the severity of fibrosis in NASH. J Gastroenterol 2007;42:573-582.
44. Wong VS, Hughes V, Trull A, Wight DG, Petrik J, Alexander GJ. Serum hyaluronic acid is a useful marker of liver fibrosis in chronic hepatitis C virus infection. J Viral Hepat 1998;5:187-192.
45. Parise ER, Oliveira AC, Figueiredo-Mendes C, Lanzoni V, Martins J, Nader H, Ferraz ML. Noninvasive serum markers in the diagnosis of structural liver damage in chronic hepatitis C virus infection. Liver Int 2006;26:1095-1099.
46. McHutchison JG, Blatt LM, de Medina M, Craig JR, Conrad A, Schiff ER, Tong MJ. Measurement of serum hyaluronic acid in patients with chronic hepatitis C and its relationship to liver histology. Consensus Interferon Study Group. J Gastroenterol Hepatol 2000;15:945-951.
47. Camacho VR, Silveira TR, Oliveira JR, Barros SG, Cerski CT. Relationship between serum concetrations of type III procollagen, hyluronic acid and histopathological findings in the liver of HCV-positive blood donors. Arq Gastroenterol 2007;44:118-122.
48. Lorenzo-Zuniga V, Bartoli R, Masnou H, Montoliu S, Morillas RM, Planas R. Serum concentrations of insulin-like growth factor-I (igf-I) as a marker of liver fibrosis in patients with chronic hepatitis C. Dig Dis Sci 2007;52:3245-3250.
49. Manolakopoulos S, Bethanis S, Liapi C, Stripeli F, Sklavos P, Margeli A, Christidou A, Katsanika A, Vogiatzakis E, Tzourmakliotis D, Theocharis S. An assessment of serum leptin levels in patients with chronic viral hepatitis: a prospective study. BMC Gastroenterol 2007;7:17.
50. Camacho VR, Silveira TR, Oliveira JR, Barros SG, Cerski CT. Relationship between serum concetrations of type III procollagen, hyluronic acid and histopathological findings in the liver of HCV-positive blood donors. Arq Gastroenterol 2007;44:118-122.
51. Leroy V, Hilleret MN, Sturm N, Trocme C, Renversez JC, Faure P, Morel F, Zarski JP. Prospective comparison of six non-invasive scores for the diagnosis of liver fibrosis in chronic hepatitis C. J Hepatol 2007;46:775-782.
52. Trocme C, Leroy V, Sturm N, Hilleret MN, Bottari S, Morel F, Zarski JP. Longitudinal evaluation of a fibrosis index combining MMP-1 and PIIINP compared with MMP-9, TIMP-1 and hyaluronic acid in patients with chronic hepatitis C treated by interferon-alpha and ribavirin. J Viral Hepat 2006;13:643-651.
53. Zheng M, Cai WM, Weng HL, Liu RH. ROC curves in evaluation of serum fibrosis indices for hepatic fibrosis. World J Gastroenterol 2002;8:1073-1076.
54. Lebensztejn DM, Sobaniec-Lotowska ME, Bauer M, Kaczmarski M, Voelker M, Schuppan D. Serum fibrosis markers as predictors of an antifibrotic effect of interferon alfa in children with chronic hepatitis B. Eur J Gastroenterol Hepatol 2005;17:843-848.
55. Lebensztejn DM, Sobaniec-Lotowska ME, Kaczmarski M, Voelker M, Schuppan D. Matrix-derived serum markers in monitoring liver fibrosis in children with chronic hepatitis B treated with interferon alpha. World J Gastroenterol 2006;12:3338-3343.
56. Tsochatzis E, Papatheodoridis GV, Hadziyannis E, Georgiou A, Kafiri G, Tiniakos DG, Manesis EK, Archimandritis AJ. Serum adipokine levels in chronic liver diseases: association of resistin levels with fibrosis severity. Scand J Gastroenterol 2008;43:1128-1136.
57. Patel K, Gordon SC, Jacobson I, Hezode C, Oh E, Smith KM, Pawlotsky JM, McHutchison JG. Evaluation of a panel of non-invasive serum markers to differentiate mild from moderate-to-advanced liver fibrosis in chronic hepatitis C patients. J Hepatol 2004;41:935-942.
58. Lieber CS, Weiss DG, Paronetto F. Value of fibrosis markers for staging liver fibrosis in patients with precirrhotic alcoholic liver disease. Alcohol Clin Exp Res 2008;32:1031-1039.
59. Forns X, Ampurdanes S, Llovet JM, Aponte J, Quinto L, Martinez-Bauer E, Bruguera M, Sanchez-Tapias JM, Rodes J. Identification of chronic hepatitis C patients without hepatic fibrosis by a simple predictive model. Hepatology 2002;36:986-992.
60. Bourliere M, Penaranda G, Renou C, Botta-Fridlund D, Tran A, Portal I, Lecomte L, Castellani P, Rosenthal-Allieri MA, Gerolami R, Ouzan D, Deydier R, Degott C, Halfon P. Validation and comparison of indexes for fibrosis and cirrhosis prediction in chronic hepatitis C patients: proposal for a pragmatic approach classification without liver biopsies. J Viral Hepat 2006;13:659-670.
61. Cacoub P, Carrat F, Bedossa P, Lambert J, Penaranda G, Perronne C, Pol S, Halfon P. Comparison of non-invasive liver fibrosis biomarkers in HIV/HCV co-infected patients: the fibrovic study--ANRS HC02. J Hepatol 2008;48:765-773.
62. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
63. Grigorescu M, Rusu M, Neculoiu D, Radu C, Serban A, Catanas M, Grigorescu MD. The FibroTest value in discriminating between insignificant and significant fibrosis in chronic hepatitis C patients. The Romanian experience. J Gastrointestin Liver Dis 2007;16:31-37.
64. Halfon P, Bacq Y, De MA, Penaranda G, Bourliere M, Ouzan D, Tran A, Botta D, Renou C, Brechot MC, Degott C, Paradis V. Comparison of test performance profile for blood tests of liver fibrosis in chronic hepatitis C. J Hepatol 2007;46:395-402.
65. Halfon P, Bourliere M, Deydier R, Botta-Fridlund D, Renou C, Tran A, Portal I, Allemand I, Bertrand JJ, Rosenthal-Allieri A, Rotily M, Sattonet C, Benderitter T, Saint Paul MC, Bonnot HP, Penaranda G, Degott C, Masseyeff MF, Ouzan D. Independent prospective multicenter validation of biochemical markers (fibrotest-actitest) for the prediction of liver fibrosis and activity in patients with chronic hepatitis C: the fibropaca study. Am J Gastroenterol 2006;101:547-555.
66. Leroy V, Halfon P, Bacq Y, Boursier J, Rousselet MC, Bourliere M, De MA, Sturm N, Hunault G, Penaranda G, Brechot MC, Trocme C, Cales P. Diagnostic accuracy, reproducibility and robustness of fibrosis blood tests in chronic hepatitis C: a meta-analysis with individual data. Clin Biochem 2008;41:1368-1376.
67. Ratziu V, Massard J, Charlotte F, Messous D, Imbert-Bismut F, Bonyhay L, Tahiri M, Munteanu M, Thabut D, Cadranel JF, Le BB, de L, V, Poynard T. Diagnostic value of biochemical markers (FibroTest-FibroSURE) for the prediction of liver fibrosis in patients with non-alcoholic fatty liver disease. BMC Gastroenterol 2006;6:6.
68. Poynard T, Imbert-Bismut F, Ratziu V, Chevret S, Jardel C, Moussalli J, Messous D, Degos F. Biochemical markers of liver fibrosis in patients infected by hepatitis C virus: longitudinal validation in a randomized trial. J Viral Hepat 2002;9:128-133.
69. Poynard T, Munteanu M, Imbert-Bismut F, Charlotte F, Thabut D, Le CS, Messous D, Thibault V, Benhamou Y, Moussalli J, Ratziu V. Prospective analysis of discordant results between biochemical markers and biopsy in patients with chronic hepatitis C. Clin Chem 2004;50:1344-1355.
70. Poynard T, Morra R, Halfon P, Castera L, Ratziu V, Imbert-Bismut F, Naveau S, Thabut D, Lebrec D, Zoulim F, Bourliere M, Cacoub P, Messous D, Munteanu M, de L, V. Meta-analyses of FibroTest diagnostic value in chronic liver disease. BMC Gastroenterol 2007;7:40.
71. Ngo Y, Munteanu M, Messous D, Charlotte F, Imbert-Bismut F, Thabut D, Lebray P, Thibault V, Benhamou Y, Moussalli J, Ratziu V, Poynard T. A prospective analysis of the prognostic value of biomarkers (FibroTest) in patients with chronic hepatitis C. Clin Chem 2006;52:1887-1896.
72. Naveau S, Raynard B, Ratziu V, Abella A, Imbert-Bismut F, Messous D, Beuzen F, Capron F, Thabut D, Munteanu M, Chaput JC, Poynard T. Biomarkers for the prediction of liver fibrosis in patients with chronic alcoholic liver disease. Clin Gastroenterol Hepatol 2005;3:167-174.
73. Myers RP, Tainturier MH, Ratziu V, Piton A, Thibault V, Imbert-Bismut F, Messous D, Charlotte F, Di M, V, Benhamou Y, Poynard T. Prediction of liver histological lesions with biochemical markers in patients with chronic hepatitis B. J Hepatol 2003;39:222-230.
74. Jacqueminet S, Lebray P, Morra R, Munteanu M, Devers L, Messous D, Bernard M, Hartemann-Heurtier A, Imbert-Bismut F, Ratziu V, Grimaldi A, Poynard T. Screening for liver fibrosis by using a noninvasive biomarker in patients with diabetes. Clin Gastroenterol Hepatol 2008;6:828-831.
75. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
76. Poynard T, Zoulim F, Ratziu V, Degos F, Imbert-Bismut F, Deny P, Landais P, El HA, Slama A, Blin P, Thibault V, Parvaz P, Munteanu M, Trepo C. Longitudinal assessment of histology surrogate markers (FibroTest-ActiTest) during lamivudine therapy in patients with chronic hepatitis B infection. Am J Gastroenterol 2005;100:1970-1980.
77. Poynard T, Munteanu M, Imbert-Bismut F, Charlotte F, Thabut D, Le CS, Messous D, Thibault V, Benhamou Y, Moussalli J, Ratziu V. Prospective analysis of discordant results between biochemical markers and biopsy in patients with chronic hepatitis C. Clin Chem 2004;50:1344-1355.
78. Myers RP, Tainturier MH, Ratziu V, Piton A, Thibault V, Imbert-Bismut F, Messous D, Charlotte F, Di M, V, Benhamou Y, Poynard T. Prediction of liver histological lesions with biochemical markers in patients with chronic hepatitis B. J Hepatol 2003;39:222-230.
79. Carvalho-Filho RJ, Schiavon LL, Narciso-Schiavon JL, Sampaio JP, Lanzoni VP, Ferraz ML, Silva AE. Optimized cutoffs improve performance of the aspartate aminotransferase to platelet ratio index for predicting significant liver fibrosis in human immunodeficiency virus/hepatitis C virus co-infection. Liver Int 2008;28:486-493.
80. Al-Mohri H, Cooper C, Murphy T, Klein MB. Validation of a simple model for predicting liver fibrosis in HIV/hepatitis C virus-coinfected patients. HIV Med 2005;6:375-378.
81. Cales P, Laine F, Boursier J, Deugnier Y, Moal V, Oberti F, Hunault G, Rousselet MC, Hubert I, Laafi J, Ducluzeaux PH, Lunel F. Comparison of blood tests for liver fibrosis specific or not to NAFLD. J Hepatol 2008.
82. Paggi S, Colli A, Fraquelli M, Vigano M, Del PP, Facciotto C, Colombo M, Ronchi G, Conte D. A non-invasive algorithm accurately predicts advanced fibrosis in hepatitis C: a comparison using histology with internal-external validation. J Hepatol 2008;49:564-571.
83. Trang T, Petersen JR, Snyder N. Non-invasive markers of hepatic fibrosis in patients co-infected with HCV and HIV: comparison of the APRI and FIB-4 index. Clin Chim Acta 2008;397:51-54.
84. Snyder N, Gajula L, Xiao SY, Grady J, Luxon B, Lau DT, Soloway R, Petersen J. APRI: an easy and validated predictor of hepatic fibrosis in chronic hepatitis C. J Clin Gastroenterol 2006;40:535-542.
85. Snyder N, Nguyen A, Gajula L, Soloway R, Xiao SY, Lau DT, Petersen J. The APRI may be enhanced by the use of the FIBROSpect II in the estimation of fibrosis in chronic hepatitis C. Clin Chim Acta 2007;381:119-123.
86. Hongbo L, Xiaohui L, Hong K, Wei W, Yong Z. Assessing routine and serum markers of liver fibrosis in CHB patients using parallel and serial interpretation. Clin Biochem 2007;40:562-566.
87. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
88. Adams LA, Bulsara M, Rossi E, DeBoer B, Speers D, George J, Kench J, Farrell G, McCaughan GW, Jeffrey GP. Hepascore: an accurate validated predictor of liver fibrosis in chronic hepatitis C infection. Clin Chem 2005;51:1867-1873.
89. Koda M, Matunaga Y, Kawakami M, Kishimoto Y, Suou T, Murawaki Y. FibroIndex, a practical index for predicting significant fibrosis in patients with chronic hepatitis C. Hepatology 2007;45:297-306.
90. Metwally MA, Zein CO, Zein NN. Predictors and noninvasive identification of severe liver fibrosis in patients with chronic hepatitis C. Dig Dis Sci 2007;52:582-588.
91. Mohamadnejad M, Montazeri G, Fazlollahi A, Zamani F, Nasiri J, Nobakht H, Forouzanfar MH, Abedian S, Tavangar SM, Mohamadkhani A, Ghoujeghi F, Estakhri A, Nouri N, Farzadi Z, Najjari A, Malekzadeh R. Noninvasive markers of liver fibrosis and inflammation in chronic hepatitis B-virus related liver disease. Am J Gastroenterol 2006;101:2537-2545.
92. Zaman A, Rosen HR, Ingram K, Corless CL, Oh E, Smith K. Assessment of FIBROSpect II to detect hepatic fibrosis in chronic hepatitis C patients. Am J Med 2007;120:280-14.
93. Patel K, Nelson DR, Rockey DC, Afdhal NH, Smith KM, Oh E, Hettinger K, Vallee M, Dev A, Smith-Riggs M, McHutchison JG. Correlation of FIBROSpect II with histologic and morphometric evaluation of liver fibrosis in chronic hepatitis C. Clin Gastroenterol Hepatol 2008;6:242-247.
94. Sebastiani G, Vario A, Guido M, Noventa F, Plebani M, Pistis R, Ferrari A, Alberti A. Stepwise combination algorithms of non-invasive markers to diagnose significant fibrosis in chronic hepatitis C. J Hepatol 2006;44:686-693.
95. Imbert-Bismut F, Ratziu V, Pieroni L, Charlotte F, Benhamou Y, Poynard T. Biochemical markers of liver fibrosis in patients with hepatitis C virus infection: a prospective study. Lancet 2001;357:1069-1075.
96. Nunes D, Fleming C, Offner G, O'Brien M, Tumilty S, Fix O, Heeren T, Koziel M, Graham C, Craven DE, Stuver S, Horsburgh CR, Jr. HIV infection does not affect the performance of noninvasive markers of fibrosis for the diagnosis of hepatitis C virus-related liver disease. J Acquir Immune Defic Syndr 2005;40:538-544.
97. Castera L, Vergniol J, Foucher J, Le BB, Chanteloup E, Haaser M, Darriet M, Couzigou P, de L, V. Prospective comparison of transient elastography, Fibrotest, APRI, and liver biopsy for the assessment of fibrosis in chronic hepatitis C. Gastroenterology 2005;128:343-350.
98. Guanabens N, Pares A, Alvarez L, Martinez de Osaba MJ, Monegal A, Peris P, Ballesta AM, Rodes J. Collagen-related markers of bone turnover reflect the severity of liver fibrosis in patients with primary biliary cirrhosis. J Bone Miner Res 1998;13:731-738.
99. Moller S, Hansen M, Hillingso J, Jensen JE, Henriksen JH. Elevated carboxy terminal cross linked telopeptide of type I collagen in alcoholic cirrhosis: relation to liver and kidney function and bone metabolism. Gut 1999;44:417-423.
100. Rosen HN, Parker RA, Greenspan SL, Iloputaife ID, Bookman L, Chapin D, Perlmutter I, Kessel B, Qvist P, Rosenblatt M. Evaluation of ability of biochemical markers of bone turnover to predict a response to increased doses of HRT. Calcif Tissue Int 2004;74:415-423.
101. Lein M, Wirth M, Miller K, Eickenberg HU, Weissbach L, Schmidt K, Haus U, Stephan C, Meissner S, Loening SA, Jung K. Serial Markers of Bone Turnover in Men with Metastatic Prostate Cancer Treated with Zoledronic Acid for Detection of Bone Metastases Progression. Eur Urol 2007.
102. Attallah AM, Toson EA, Shiha GE, Omran MM, bdel-Aziz MM, El-Dosoky I. Evaluation of serum procollagen aminoterminal propeptide III, laminin, and hydroxyproline as predictors of severe fibrosis in patients with chronic hepatitis C. J Immunoassay Immunochem 2007;28:199-211.
103. Ulrich D, Noah EM, von HD, Pallua N. TIMP-1, MMP-2, MMP-9, and PIIINP as serum markers for skin fibrosis in patients following severe burn trauma. Plast Reconstr Surg 2003;111:1423-1431.
104. Farkkila M, Rautiainen H, Karkkainen P, Karvonen AL, Nurmi H, Niemela O. Serological markers for monitoring disease progression in noncirrhotic primary biliary cirrhosis on ursodeoxycholic acid therapy. Liver Int 2008;28:787-797.
105. Guechot J, Poupon RE, Giral P, Balkau B, Giboudeau J, Poupon R. Relationship between procollagen III aminoterminal propeptide and hyaluronan serum levels and histological fibrosis in primary biliary cirrhosis and chronic viral hepatitis C. J Hepatol 1994;20:388-393.
106. Klappacher G, Franzen P, Haab D, Mehrabi M, Binder M, Plesch K, Pacher R, Grimm M, Pribill I, Eichler HG, . Measuring extracellular matrix turnover in the serum of patients with idiopathic or ischemic dilated cardiomyopathy and impact on diagnosis and prognosis. Am J Cardiol 1995;75:913-918.
107. Imbert-Bismut F, Ratziu V, Pieroni L, Charlotte F, Benhamou Y, Poynard T. Biochemical markers of liver fibrosis in patients with hepatitis C virus infection: a prospective study. Lancet 2001;357:1069-1075.
108. Suzuki,K., Enghild,J.J., Morodomi,T., Salvesen,G., and Nagase,H. 1990. Mechanisms of activation of tissue procollagenase by matrix metalloproteinase 3 (stromelysin). Biochemistry 29:10261-10270.
109. Lijnen,H.R. 2001. Plasmin and matrix metalloproteinases in vascular remodeling. Thromb. Haemost. 86:324-333.
110. Tam LS, Gu J, Yu D. Pathogenesis of ankylosing spondylitis. Nat Rev Rheumatol 2010 Jul;6(7):399-405.
111. Braun J, Pincus T. Mortality, course of disease and prognosis of patients with ankylosing spondylitis. Clin Exp Rheumatol 2002 Nov;20(6 Suppl 28):S16-S22.
112. Kumar VAAKFN. Tissue renewal and repair: regeneration, healing, and fibrosis. Pathologic basis of disease.Philadelphia, Pennsylvania, USA: Elsevier Saunders, 2005. 87-118.
113. Schuppan D, Ruehl M, Somasundaram R, Hahn EG. Matrix as a modulator of hepatic fibrogenesis. Semin Liver Dis 2001 Aug;21(3):351-372.
114. Lochter A, Bissell MJ. An odyssey from breast to bone: multi-step control of mammary metastases and osteolysis by matrix metalloproteinases. APMIS 1999 Jan;107(1):128-136.
115. Karsdal MA, Madsen SH, Christiansen C, Henriksen K, Fosang AJ, Sondergaard BC. Cartilage degradation is fully reversible in the presence of aggrecanase but not matrix metalloproteinase activity. Arthritis Res Ther 2008;10(3):R63.
116. Karsdal MA, Henriksen K, Leeming DJ, Mitchell P, Duffin K, Barascuk N, et al. Biochemical markers and the FDA Critical Path: how biomarkers may contribute to the understanding of pathophysiology and provide unique and necessary tools for drug development. Biomarkers 2009 May;14(3):181-202.
117. Bay-Jensen AC, Hoegh-Madsen S, Dam E, Henriksen K, Sondergaard BC, Pastoureau P, et al. Which elements are involved in reversible and irreversible cartilage degradation in osteoarthritis? Rheumatol Int 2010 Feb;30(4):435-442.
118. Zhen EY, Brittain IJ, Laska DA, Mitchell PG, Sumer EU, Karsdal MA, et al. Characterization of metalloprotease cleavage products of human articular cartilage. Arthritis Rheum 2008 Aug;58(8):2420-2431.
119. Boeker KH, Haberkorn CI, Michels D, Flemming P, Manns MP, Lichtinghagen R. Diagnostic potential of circulating TIMP-1 and MMP-2 as markers of liver fibrosis in patients with chronic hepatitis C. Clin Chim Acta 2002 Feb;316(1-2):71-81.
120. Hemmann S, Graf J, Roderfeld M, Roeb E. Expression of MMPs and TIMPs in liver fibrosis - a systematic review with special emphasis on anti-fibrotic strategies. J Hepatol 2007 May;46(5):955-975.
121. Kirimlioglu H, Kirimlioglu V, Yilmaz S. Expression of matrix metalloproteinases 2 and 9 in donor liver, cirrhotic liver, and acute rejection after human liver transplantation. Transplant Proc 2008 Dec;40(10):3574-3577.
122. Schaller S, Henriksen K, Hoegh-Andersen P, Sondergaard BC, Sumer EU, Tanko LB, et al. In vitro, ex vivo, and in vivo methodological approaches for studying therapeutic targets of osteoporosis and degenerative joint diseases: how biomarkers can assist? Assay Drug Dev Technol 2005 Oct;3(5):553-580.
123. Wenstrup RJ, Florer JB, Brunskill EW, Bell SM, Chervoneva I, Birk DE. Type V collagen controls the initiation of collagen fibril assembly. J Biol Chem 2004 Dec 17;279(51):53331-53337.
124. Symoens S, Renard M, Bonod-Bidaud C, Syx D, Vaganay E, Malfait F, et al. Identification of binding partners interacting with the alpha1-N-propeptide of type V collagen. Biochem J 2010 Dec 22;433(2):371-381.
125. Berendsen AD, Bronckers AL, Smit TH, Walboomers XF, Everts V. Collagen type V enhances matrix contraction by human periodontal ligament fibroblasts seeded in three-dimensional collagen gels. Matrix Biol 2006 Oct;25(8):515-522.
126. Murasawa Y, Hayashi T, Wang PC. The role of type V collagen fibril as an ECM that induces the motility of glomerular endothelial cells. Exp Cell Res 2008 Dec 10;314(20):3638-3653.
127. Vassiliadis E, Veidal SS, Simonsen H, Larsen DV, Vainer B, Chen X, et al. Immunological detection of the type V collagen propeptide fragment, PVCP-1230, in connective tissue remodeling associated with liver fibrosis. Biomarkers 2011 May 25.
128. Birk DE, Fitch JM, Babiarz JP, Linsenmayer TF. Collagen type I and type V are present in the same fibril in the avian corneal stroma. J Cell Biol 1988 Mar;106(3):999-1008.
129. Wenstrup RJ, Florer JB, Willing MC, Giunta C, Steinmann B, Young F, et al. COL5A1 haploinsufficiency is a common molecular mechanism underlying the classical form of EDS. Am J Hum Genet 2000 Jun;66(6):1766-1776.
130. Schwarze U, Atkinson M, Hoffman GG, Greenspan DS, Byers PH. Null alleles of the COL5A1 gene of type V collagen are a cause of the classical forms of Ehlers-Danlos syndrome (types I and II). Am J Hum Genet 2000 Jun;66(6):1757-1765.
131. Michalickova K, Susic M, Willing MC, Wenstrup RJ, Cole WG. Mutations of the alpha2(V) chain of type V collagen impair matrix assembly and produce ehlers-danlos syndrome type I. Hum Mol Genet 1998 Feb;7(2):249-255.
132. Gefter ML, Margulies DH, Scharff MD. A simple method for polyethylene glycol-promoted hybridization of mouse myeloma cells. Somatic Cell Genet 1977 Mar;3(2):231-236.
133. Segovia-Silvestre T, Reichenbach V, Fernandez-Varo G, Vassiliadis E, Barascuk N, Morales-Ruiz M, Karsdal MA, Jimenez W. Circulating CO3-610, a degradation product of collagen III, closely reflects liver collagen and portal pressure in rats with fibrosis. Fibrogenesis Tissue Repair 2011; 4: 19
134. Clarià J , Jimenez W. Renal dysfunction and ascites in carbon tetrachloride-induced cirrhosis in rats. 1999; 379-96
135. Munoz-Luque J, Ros J, Fernandez-Varo G, Tugues S, Morales-Ruiz M, Alvarez CE, Friedman SL, Arroyo V, Jimenez W. Regression of fibrosis after chronic stimulation of cannabinoid CB2 receptor in cirrhotic rats. J Pharmacol Exp Ther 2008; 324: 475-83
136. Veidal SS, Karsdal MA, Nawrocki A, Larsen MR, Dai Y, Zheng Q, Hagglund P, Vainer B, Skjot-Arkil H, Leeming DJ. Assessment of proteolytic degradation of the basement membrane: a fragment of type IV collagen as a biochemical marker for liver fibrosis. Fibrogenesis Tissue Repair 2011; 4: 22
137. Veidal SS, Vassiliadis E, Barascuk N, Zhang C, Segovia-Silvestre T, Klickstein L, Larsen MR, Qvist P, Christiansen C, Vainer B, Karsdal MA. Matrix metalloproteinase-9-mediated type III collagen degradation as a novel serological biochemical marker for liver fibrogenesis. Liver Int 2010; 30: 1293-304
138. Clarkson TB, Prichard RW, Morgan TM, Petrick GS, Klein KP. Remodeling of coronary arteries in human and nonhuman primates. JAMA 1994; 271: 289-94
139. Conroy RM, Pyorala K, Fitzgerald AP, Sans S, Menotti A, De BG, De BD, Ducimetiere P, Jousilahti P, Keil U, Njolstad I, Oganov RG, Thomsen T, Tunstall-Pedoe H, Tverdal A, Wedel H, Whincup P, Wilhelmsen L, Graham IM. Estimation of ten-year risk of fatal cardiovascular disease in Europe: the SCORE project. Eur Heart J 2003; 24: 987-1003

## Claims

1. A method of bioassay comprising, conducting an immunoassay to measure neo-epitope containing protein fragments naturally present in a biofluid sample, wherein said immunoassay is conducted by a method comprising:
contacting protein fragments naturally present in said sample with an immunological binding partner specifically reactive with a neo-epitope formed by cleavage of a protein by a proteinase and measuring the extent of binding of peptide fragments to said immunological binding partner to measure therein protein fragments comprising said neo-epitope, wherein said immunological binding partner is specifically reactive with the following sequence at the N terminal of a peptide: KTFGKM

2. A method as claimed in claim 1, wherein said detection of binding is quantative.

3. A method as claimed in claim 1, wherein the amount of binding is compared to control values established for populations of healthy individuals and of individuals **characterised by** a fibrotic disease or by an inflammatory condition.

4. A method as claimed in claim 1, conducted as a competition assay such that peptides in said sample compete for binding to the antibody or antibody fragment with a known concentration of a binding agent which binds said antibody or antibody fragment.

## Patentansprüche

1. Bioassayverfahren, das das Durchführen eines Immunassays zum Messen von neoepitophaltigen Proteinfragmenten, die in einer Bioflüssigkeitsprobe natürlich vorhanden sind, umfasst, wobei der Immunassay mit einem Verfahren durchgeführt wird, das umfasst:
Inkontaktbringen von Proteinfragmenten, die in der Probe natürlich vorhanden sind, mit einem immunologischen Bindungspartner, der mit einem Neoepitop spezifisch reaktiv ist, das durch Spalten eines Proteins durch eine Proteinase gebildet wird, und Messen des Ausmaßes der Bindung von Peptidfragmenten an den immunologischen Bindungspartner zum Messen von Proteinfragmenten darin, die das Neoepitop umfassen, wobei der immunologische Bindungspartner mit der folgenden Sequenz am N-Terminus eines Peptids spezifisch reaktiv ist: KTFGKM.

2. Verfahren nach Anspruch 1, wobei der Bindungsnachweis quantitativ ist.

3. Verfahren nach Anspruch 1, wobei die Bindungshöhe mit Kontrollwerten verglichen wird, die für Populationen von gesunden Individuen und Individuen, die durch eine fibrotische Krankheiten oder einen Entzündungszustand gekennzeichnet sind, generiert wurden.

4. Verfahren nach Anspruch 1, das als Konkurrierungsassay durchgeführt wird, so dass Peptide in der Probe um die Bindung an den Antikörper oder das Antikörperfragment mit einer bekannten Konzentration eines an den Antikörper oder das Antikörperfragment bindenden Bindemittels konkurrieren.

## Revendications

1. Méthode de bioessai comprenant : conduire un immunoessai pour mesurer des fragments de protéine contenant un néo-épitope naturellement présents dans un échantillon de fluide biologique, dans laquelle ledit immunoessai est conduit par une méthode comprenant :
mettre en contact des fragments de protéine naturellement présents dans ledit échantillon avec un partenaire de liaison immunologique capable de réagir de façon spécfique avec un néo-épitope formé par clivage d'une protéine par une protéinase et mesurer le degré de liaison de fragments peptidiques audit partenaire de liaison immunologique pour y mesurer les fragments de protéine comprenant ledit néo-épitope, ledit partenaire de liaison immunologique étant capable de réagir de façon spécifique avec la séquence suivante à l'extrémité N-terminale d'un peptide :
KTFGKM.

2. Méthode selon la revendication 1, dans laquelle ladite détection de liaison est quantitative.

3. Méthode selon la revendication 1, dans laquelle la quantité de liaison est comparée à des valeurs témoins établies pour des populations d'individus en bonne santé et d'individus **caractérisés par** une maladie fibrotique ou par un état inflammatoire.

4. Méthode selon la revendication 1, conduite en tant qu'essai par compétition de telle sorte que les peptides dans ledit échantillon entrent en compétition pour la liaison à l'anticorps ou au fragment d'anticorps avec une concentration connue d'un agent de liaison qui se lie audit anticorps ou fragment d'anticorps.
